# EUROPEAN PATENT APPLICATION

(11) **EP 3 165 240 A1**
(43) Date of publication of application: **10.05.2017**
(21) Application number: 15193284.5
(22) Date of filing: 05.11.2015
(51) Int. Cl.: A61L 27/48

(54) **NATURAL POLYMER-DERIVED SCAFFOLD MATERIAL AND METHODS FOR PRODUCTION THEREOF**

(71) Applicant: ECOLE POLYTECHNIQUE FEDERALE DE LAUSANNE (EPFL), 1015 Lausanne (CH)
(72) Inventor: HUBBELL, Jeffrey Alan, Chicago, IL 60615 (US); FREY, Peter, 1066 EPALINGES (CH); LUTOLF, Matthias, 1131 TOLOCHENAZ (CH); VARDAR, Elif, 1026 ECHANDENS-DENGES (CH); LARSSON, Hans Mattias, 1004 LAUSANNE (CH); BALET, Eva-Maria, 2088 CRESSIER (CH); PINNAGODA, Kalitha, 1806 ST-LEGIER (CH); ALLAZETTA, Simone, 1024 ECUBLENS (CH)
(74) Representative: Grosfillier, Philippe

(57) **Abstract**

The invention relates to a scaffold material comprising a carrier and embedded microbeads for use in tissue engineering applications such as soft tissues therapeutic treatment. The scaffold provides a short-term bulking effect coupled with a long-term functional activity. Both the carrier and the microbeads are substantially composed of natural or extracellular matrix-derived polymers, and the beads can comprise homogeneously distributed active agents, providing a regulated agent release along time. An aspect of the invention relates to a method for producing the microbeads of the invention by using an expressly designed microfluidic chip.

## Description

### Technical Field

The present invention lies in the field of tissue engineering. More particularly, it relates to a novel kind of microbead-based biomaterial obtained from extracellular matrix (ECM) components, as well as to methods for producing thereof.

### Background Art

Collagen is the most abundant structural extracellular matrix protein in the human body, and therefore is widely used as a scaffold component for tissue engineering applications. Different methods have been developed to overcome its weak mechanical properties and to create scaffolds with appropriate strength. One option is the lyophilization (freeze-drying) of an aqueous collagen solution followed by chemical cross-linking (Macomber L 2005). Another possibility is freeze-drying of a collagen gel followed by thermal dehydration cross-linking, or the simple removal of excess water from a collagen gel, a process called plastic compression (Shimizu 1999) (Brown 2005). Furthermore, collagen hybrid structures have been reported where a synthetic polymer component provided the mechanical support (Kawazoe N 2010) (Lu H 2012).

Another widely used biomaterial is fibrin. Fibrin is an integral component of the biologically active clot within healing wounds. It is used as a sealant in several clinical applications. Furthermore, fibrin is also a promising scaffold material due to its inherent biological activity arising from its integrin and growth factor binding sites. It has been successfully used in several tissue engineering applications including bone, cardiac tissue, cartilage, muscle, and neural tissue (Zhou H 2011; Jockenhoevel S 2011; Eyrich D 2007; Page RL 2011; Willerth SM 2006).

Additionally, hybrid scaffolds made of collagen and fibrin are reported. Studies were conducted to understand the co-gelation of both proteins as well as the resulting microstructure in function of different protein concentrations and fibrin-polymerizing enzymes (Rowe SL 2009; Lai VK 2012). Fibrin-collagen scaffolds were mainly used for vascular tissue engineering. Either the in vitro microvascular network formation of endothelial cells within gels was analysed or collagen-fibrin mixtures were constructed in a three-dimensional tubular shape and seeded with human aortic smooth muscle cells with the goal to develop small-diameter tissue-engineered vascular grafts (Park YK 2014; Cummings CL 2003; Rao RR 2012). Furthermore, single-walled carbon nanotubes were embedded into collagen-fibrin matrices to create electrically conductive scaffold materials (Voge CM 2008).

One tissue regeneration strategy is based on scaffold-based delivery of signalling molecules such as growth factors. Growth factors affect cell migration, proliferation, and differentiation. They have a very local action under physiological conditions due to their short half-lives and slow diffusion. A number of growth factors have already been tested in clinical trials including vascular endothelial growth factor (VEGF). VEGF was directly injected into the body to induce neovascularization. Phase I trials reported promising results (Schumacher B 1998). However, the larger phase II trials did not show the beneficial effect for patients (Simons M 2002). The disappointing clinical results show the need for the development of more efficient growth factor delivery strategies. One promising strategy is based on natural polymer matrices as delivery substrate for growth factors. Since the matrix is also affecting the cell fate, natural polymer matrices are advantageous over synthetic polymer matrices because they are able to interact at the molecular level, are biocompatible, and cause only minimal chronic infection. However, it is hard to control some of their physical parameters such as degradation rate.

One natural polymer used for growth factor delivery is fibrin. Various approaches have been investigated to alter the release kinetic of growth factors from fibrin matrices. One way was to slow down the fibrin degradation rate by incorporation of anti-fibrinolytic substances like aprotinin (Sacchi V 2014). Furthermore, the alteration of the fibrinogen and thrombin concentration affected the growth factor release kinetics. As the concentration of thrombin increased, the release of basic fibroblast growth factor 2 (FGF-2) was delayed due to the higher level of matrix cross-linking (Jeon O 2005). Similarly, the porosity of the matrix could be increased by increasing the concentration of fibrinogen; thus inhibiting the passive diffusion of the growth factor (Jeon O 2005). A more efficient way to slow down the passive diffusion of growth factors from fibrin gels, was the addition of heparin to the matrix. Many growth factors display an affinity towards heparin. Due to its increased size, the heparin-growth factor complex diffuses more slowly through the fibrin matrix, delaying its release. This allows the fibrin matrix to act as a growth factor reservoir and encouraging tissue regeneration (Han H 2010).

Compared to physical incorporation, covalent binding of growth factors to the matrix sustains their release over a longer time period. Therefore, a factor XIIIa substrate, derived from the plasmin α2 substrate sequence, is fused onto the N-terminus of the growth factor, allowing the covalent binding of the growth factor to the fibrin network during normal factor XIIIa-mediated polymerization (Ehrbar M 2008). Another strategy comprises the fusion of the kringel binding domain to the growth factor (Zhao W 2009). Plasminogen contains 5 different kringel domains all of which have a high affinity towards fibrin. Furthermore, recombinant growth factors have been designed that not only allow covalent binding to the fibrin matrix but also provide a cell-mediated release. This mechanism provides a variable rate of growth factor release dependent on local cellular activity (Peterson AW 2014). Therefore, a factor XIIIa substrate and a cleavage site (i.e. plasmin α2 degradation substrate sequence) were introduced to the N-terminus of the growth factor (Sakiyama-Elbert SE 2001). Recently, recombinant growth factors were reported that showed a super-affinity to extracellular matrix proteins including fibrin and collagen (Martino M 2014). The super-affinity to the extracellular matrix was due to the fusion of placenta growth factor-2 derived domain (PIGF-2123-144) to the growth factor. Thus, many ways exist to efficiently deliver growth factors from scaffolds made from extracellular matrix proteins.

Collagen in the form of gels and sponges has also been widely used as delivery system for growth factors. Most of the collagen based delivery systems just trap the growth factor inside the collagen fibrils and the release depends on micro dimension of the collagen fibers and growth factors. A major drawback to this delivery strategy is that the time course is not well controlled. Typically, there is a burst release within the first few hours and the total release time is relatively short. In order to overcome these problems, strategies have also been developed for collagen-based delivery systems to provide a more controlled and long-term growth factor delivery.

As described for the fibrin matrices, heparin was also used in collagen scaffolds to enhance growth factor delivery. Heparin was cross-linked to collagen using a method called EDC chemistry (i.e. crosslinking proteins and peptides via a carboxyl-to-amine link). Growth factors, presenting a heparin-binding domain were efficiently retained by those collagen-heparin scaffolds (Wu JM 2011; Sun B 2009). EDC chemistry was also used to covalently immobilize VEGF onto porous collagen scaffolds (Chiu LL 2011). Further modifications realized on collagen scaffolds to enhance growth factor binding included the addition of glycosaminoglycans via EDC chemistry or the addition of sulfhydryl groups to collagen scaffolds (Mullen LM 2010; He Q 2011). Glycosaminoglycans are polyanionic and therefore bind growth factors. The presence of sulfhydryl groups and the use of the cross-linker reagent sulfo-SMCC allowed the conjugation of growth factors without affecting their biological activity. Another possibility to irreversibly cross-link growth factors to collagen-based scaffolds was described (Niger C 2013). Transglutaminase, catalyzing the formation of covalent lysine-amide bonds between individual protein strands, was used to bind biologically active transforming growth factor (TGF) onto collagen type II coated poly(lactic acid) nanofibrous scaffolds. A widely used method for efficient growth factor delivery from collagen scaffolds is the use of collagen-binding domains. Several collagen bindings domains were identified such as TKKTLRT derived from collagenase or WREPSFCALS derived from Willebrand's factor. These collagen-binding domains were fused to the N-terminus of several growth factors; thus enhancing the retention of growth factors within collagen scaffolds (Ma F 2014; Tan Q 2014).

As summarized above, both collagen and fibrin are used as delivery systems for growth factors. In terms of regeneration efficiency, both matrices were able to achieve good results. But where fibrin excels, is in its ability to be functionalized during its polymerization process. This allows the modification and fine-tuning of growth factor release.

As an alternative to traditional three-dimensional scaffolds, polymeric micro-beads were proposed as cell carrier in suspension cultures in vitro or as microenvironment to guide cell differentiation. Furthermore, micro-beads were employed for cell and drug delivery in surgical applications. Cells within micro-beads were protected against mixing and injection forces. After injection, cells delivered on micro-beads were immediately exposed to nutrients in the interstitial fluid, improving cell proliferation and viability. However, only a few publications reported pure fibrin micro-beads or fibrin micro-beads combined with other natural polymers. The most commonly used method to prepare fibrin micro-beads was an oil-emulsion technique performed at temperatures of 60-80°C (Gorodetsky R 1999; Gerard Marx 2002), but these conditions does not allow the incorporation of cells or bioactive molecules within the fibrin micro-beads.

Cell-friendly fabrication methods were described for collagen-fibrin and alginate-fibrin micro-beads. Cell-seeded collagen-fibrin micro-beads were prepared by suspending cells in a solution of collagen and fibrin. This cell suspension was then emulsified in a cold silicon bath by controlled stirring. Gelation of the collagen-fibrin matrix was initiated by increasing the temperature up to 37°C resulting in spheroidal micro-beads with encapsulated cells (Peterson AW 2014). The obtained collagen-fibrin micro-beads had a diameter of 205±71 µm and their stability was ensured by glyoxal induced crosslinking. Alginate-fibrin micro-beads were produced by preparing a solution of alginate, cells, and fibrinogen. This solution was pipetted drop by drop into a polymerization solution made of calcium chloride and thrombin (Perka C 2001). Alginate could be extracted from alginate-fibrin micro-beads by sodium citrate resulting in pure fibrin micro-beads. All these approaches are conceived and optimized for the culture and transplantation of cells embedded within the beads, and allow a very limited freedom concerning the tailoring of the features of the final product (e.g. size of the beads, concentration of the used materials, eventual ratio thereof and so forth).

Injectable biomaterials were developed as an attractive alternative to surgical procedures for the treatment of stress urinary incontinence, vesicoureteral reflux, esophageal reflux and fecal incontinence. Their advantages lie in the minimally invasive nature and the low complication rates. However, the development of an ideal urethral bulking agent remains a persistent challenge owing to recurrent clinical concerns over long-term efficacy, cost effectiveness, and patient safety. Currently used bulking agents comprise silicone particles (Macroplastique™), calcium hydroxylapatite (Coaptite™), porcine dermis (Permacol™), glutaraldehyde cross-linked bovine collagen (Contigen™), carbon beads (Durasphere™), and polyacrylamide hydrogel (Bulkamid®). The most frequently applied urethral bulking agent is Contigen™ having a cure rate of 53% (Corcos J 2005). Higher success rates were reported for Coapite™ and Durasphere™, but both of them showed also higher occurrence of postoperative transient urinary retention (Lightner DJ 2011; Mayer RD 2007). The current research aims to develop a bulking formulation that provides a bulking effect (passive effect) and stimulates regeneration of smooth muscle tissue (bioactive therapy), thus ensuring the long-term efficiency of the injectable biomaterial. Narrowing of the urethra and regeneration of smooth muscle around the urethra was seen one month after injection of polycaprolactone/Pluronic F127 porous beads with immobilized basic fibroblast growth factor or vascular endothelial growth factor in a rat model (Kim IG 2011). A dual growth factor-loaded hydrogel system made of growth factor-loaded heparin/pluronic hydrogel and growth factor-loaded gelatin-polyethylene glycol-tyramine hydrogel showed also promising results (Oh SH 2015). It created a passive bulking effect and stimulated nerve and smooth muscle regeneration at the applied urethra site.

Despite all the advancements in the biomaterial field for tissue engineering applications, there is still a need for scaffold materials to be possibly used as bulking agents that confer a short term bulking effect and a long-term functional repair, avoiding at the same time inflammation and scar tissue formation once used in a tissue regeneration setting. Moreover, such bulking agent should preferably be injectable for a minimally invasive therapeutic approach. In this context, what is still further lacking in the field is a quick, reliable and tuneable method for producing such bulking agents.

### Summary of invention

Bearing in mind all the drawbacks of the prior art, and in order to tackle and overcome them, the present inventors developed an original method for producing under mild conditions a new biomaterial consisting of or comprising natural polymer-derived micro-beads, said method allowing incorporation of bioactive molecules within the beads matrix to enhance the tissue regeneration capacity. These micro-beads with or without bioactive molecules incorporated therein can be embedded within a second natural polymer-based scaffold, such as extracellular matrix (ECM)-based scaffold, substantially acting as a bulking agent.

The polymer micro-beads, and other scaffolds comprising them, can be implanted or preferably injected into a subject in need thereof, thus serving as scaffold for tissue engineering applications or as an agent in e.g. surgical or cosmetic procedures, thus providing a large field of applications. The so obtained scaffold material can provide immediate short-term bulking effect, by e.g. increasing the resistance for tubular body structures (i.e. urethra, ureter, esophagus, rectum) and thus treating reflux and incontinence diseases, while also inducing a long-term functional repair of the damaged tissues.

The manufacturing method involves the use of a microfluidic chip expressly designed for providing micrometer-sized beads of desired, tuneable characteristics, with precisely controlled dimension and physico-chemical properties, in a quick and trustworthy way, said method being at the same time compatible with the very nature of the elements composing the micro-beads, in particular bioactive agents that could be coupled/embedded therein. One of the key features of the invention relies in the fact that the inventors were able to produce natural polymer-derived microbeads able to incorporate, during the manufacturing process, active agents within them in a homogeneous manner. For that, a mild, controllable temperature-dependent polymerization process, that does not alter the physico-chemical properties of the embedded molecules, was used.

It is therefore an object of the present invention to provide a scaffold material for use in tissue engineering, characterized in that it comprises a plurality of polymeric microbeads embedded within a polymeric carrier, wherein the microbeads and the carrier are substantially composed of the same or different natural polymeric material or extracellular matrix-derived polymeric material.

In one embodiment, the microbeads have a diameter comprised between 10 and 1000 µm, more preferably between 80 and 500 µm, even more preferably between 100 and 200 µm.

In one embodiment, the average molecular weight of the polymeric material substantially composing the microbeads and/or the carrier is comprised between about 1 and 1000 kDa, preferably between 50 and 600 kDa.

In one embodiment, the microbeads polymer density is of at least 1 mg/mL.

In one embodiment, the carrier polymer density is of at least 1 mg/mL.

In one embodiment, the microbeads volume constitutes in between 1 to 99 % of the volume of the scaffold material, preferably between 20 to 60 %.

In one embodiment, the microbeads' in vitro degradation rate can be altered by crosslinking, using inhibitor molecules, increasing polymer density, changing its porosity, its molecular weight distribution and its crystallinity.

In one embodiment, the carrier degradation rate upon in vivo application is dependent on the stabilization of the carrier polymer (i.e. use of crosslinking agents, higher protein concentrations, and implant site etc).

In one embodiment, the scaffold material is characterized in that it is a soft material.

In one embodiment, the scaffold material is characterized in that it is flowable and injectable through a cannula or a needle.

In a particular embodiment, the polymeric carrier is substantially composed of collagen. In another particular embodiment, the microbeads are substantially composed of fibrin.

In one embodiment, the microbeads are characterized in that they comprise a bioactive molecule embedded therein. In a preferred embodiment, the bioactive molecule is homogeneously embedded within a microbead.

In one embodiment, the bioactive molecule is released from the microbeads upon degradation of this latter in a substantially linear fashion.

A further object of the present invention relates to a pharmaceutical composition comprising the above-mentioned scaffold material.

Still a further object of the present invention relates to the above-mentioned scaffold material and/or pharmaceutical composition for use in the treatment or prevention of a pathological condition in a subject.

In a preferred embodiment, the pathological condition is a condition of a soft tissue and/or organ of the subject. In one embodiment, the tissue or organ is a urinary tract component (including kidney), a blood vessel, a muscle, a cartilage, skin, liver, a cornea, trachea, esophagus, heart, pharynx or inner ear tissue.

A further object of the present invention relates to a method of manufacturing microbeads substantially composed of a natural polymeric material or an extracellular matrix-derived polymeric material, wherein the polymerization of said polymeric material is a temperature-dependent polymerization, said method comprising the steps of:
a) providing a microfluidic chip comprising:
   - at least two sample reservoirs operatively connected with a pressure source adapted to apply a positive pressure thereon, at least one of said reservoirs being intended for containing an aqueous solution comprising a precursor of the polymeric material substantially composing the microbeads and at least one of said reservoirs being intended for containing an aqueous solution comprising a polymerization catalyser;
   - at least one channel operatively connected with each of the said sample reservoirs through their inlets;
   - a mixing point operatively connecting the outlets of each of said channels;
   - at least one microsized channel stemming from said mixing point;
   - at least one organic phase reservoir operatively connected with a pressure source adapted to apply a positive pressure thereon, said reservoir being intended for containing an organic solution;
   - at least one microsized channel operatively connected with each of the said organic phase reservoirs through its inlet, this microsized channel intersecting the microsized channel stemming from the mixing point in a beads-forming point;
   - a focusing element stemming from said beads-forming point adapted so to canalize the microbeads; and
   - a microbeads reservoir operatively connected with both the focusing element and means for regulating the temperature in the reservoir;
b) providing a precursor of the polymeric material substantially composing the microbeads into at least one of the sample reservoirs and a polymerization catalyser into at least one of the sample reservoirs;
c) applying a positive pressure on the sample reservoirs so that the precursor of the polymeric material and the catalyser are allowed to flow into the channels operatively connected therewith and to mix in the mixing point;
d) applying a positive pressure on the organic phase reservoirs so that the organic solution is allowed to flow into the microsized channel operatively connected therewith;
e) collecting the precursor microbeads obtained through the steps a) to d) into the microbeads reservoir;
f) regulating the temperature in the microbeads reservoir; and
g) let the precursor microbeads into the reservoir for a sufficient time for permitting a temperature-dependent polymerization thereof,
   wherein step c) and step d) are interchangeable.

In one embodiment, the microfluidic chip is a T-junction, Y-junction or flow focusing microfluidic chip.

In one embodiment, the method is characterized in that the precursor of the polymeric material is functionalized.

In one embodiment, the method is characterized in that at least one sample reservoir further comprises a bioactive molecule. In a preferred embodiment, the bioactive molecule is a macromolecule.

In one embodiment, the method is characterized in that the microfluidic chip further comprises at least one sample reservoir comprising only a bioactive molecule and no polymer material precursor or catalyser.

In one embodiment, the method is characterized in that the temperature of polymerization of the polymeric material does not alter the physico-chemical properties or the activity of the bioactive molecule.

In one embodiment, the method is characterized in that the bioactive molecule is eventually embedded into the microbeads. In a preferred embodiment, the bioactive molecule is homogeneously embedded within a microbead.

In one embodiment, the method is characterized in that the mixing point of the microfluidic chip comprises or consists of a chamber.

In one embodiment, the method is characterized in that each pressure source of the microfluidic chip acting on the reservoirs is individually addressable.

In a preferred embodiment, the method is characterized in that the catalyser is an enzyme.

In a still preferred embodiment, the method is characterized in that the polymeric material precursor is fibrinogen and the catalyser is a mixture of thrombin and Factor XIIIa.

In a preferred embodiment, the method is characterized in that the microbeads have a diameter comprised between 10 and 1000 µm, more preferably between 80 and 500 µm, even more preferably between 100 and 200 µm.

Another object of the present invention relates to microbeads obtained through the above-mentioned method.

In one embodiment, the microbeads are characterized in that they are further functionalized on their surface and/or their core.

### Brief description of drawings

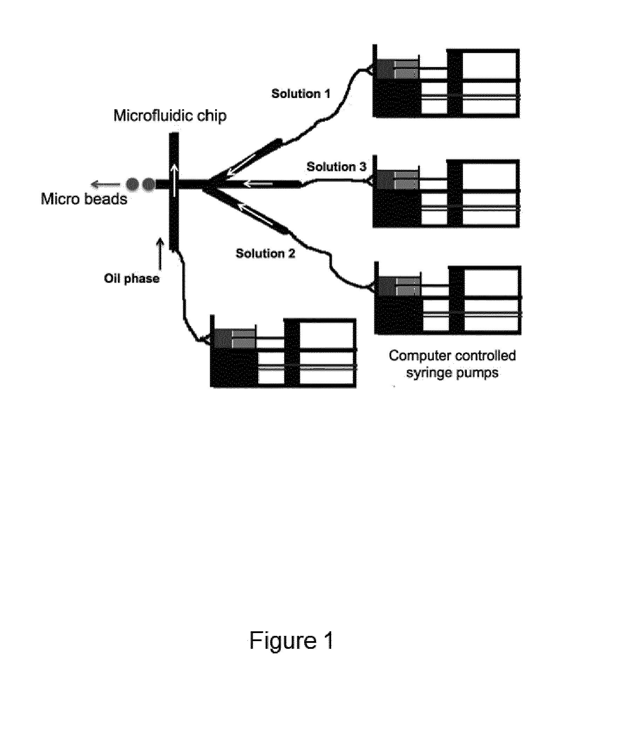

Figure 1 shows a scheme of an embodiment of the microfluidic chip according to the invention. Solution 1 refers to the polymer precursor aqueous solution; solution 2 refers to the catalyser aqueous solution; solution 3 refers to the bioactive molecules solution. The depicted microfluidic works in a flow focusing design setting.

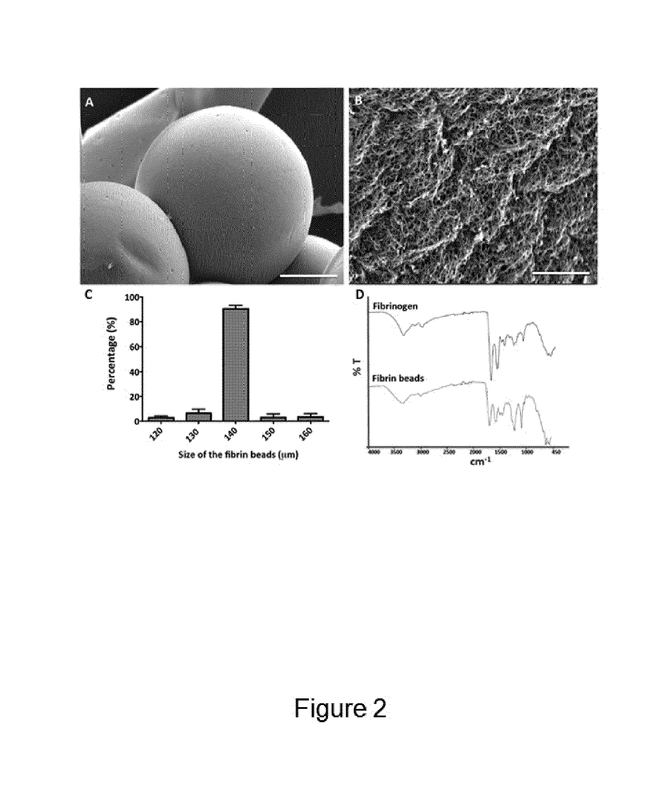

Figure 2 shows surface morphology, size determination and spectroscopic analysis of fibrin beads. (A) and (B) Scanning electron microscopy (SEM) images of fibrin beads. (C) Size distribution of fibrin beads. Pictures of fibrin beads were taken under a bright-field microscope. The pictures were further analyzed using the software ImageJ to measure the diameter of the beads. (D) FT-IR spectra of fibrinogen (black line) and fibrin beads (green line). The scanning range was from 4000 to 650 cm⁻¹ with a resolution of 4 cm⁻¹. Scale bars represent (A) 50 µm and (B) 20 µm.

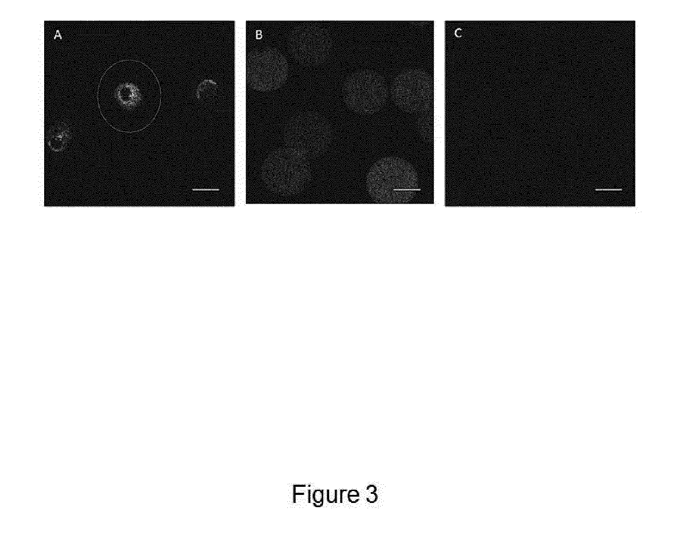

Figure 3 shows the distribution of insulin like growth factor-1 (IGF-1) within fibrin microbeads. Fibrin microbeads were incubated overnight with antihuman IGF-1 antibodies followed by the incubation with the corresponding FITC-conjugated secondary antibodies and visualized under a fluorescent microscope. (A) α₂PI₁₋₈-IGF-1 coated fibrin microbeads. (B) α₂PI₁₋₈-IGF-1 conjugated microbeads. (C) Fibrin microbeads containing no α₂PI₁₋₈-IGF-1 (control). Scale bars represent 100 µm. The term "IGF-1-conjugated microbeads" or "microbeads conjugated with IGF-1" refers to microbeads where IGF-1 was incorporated during bead production. Therefore, IGF-1 is homogenously distributed throughout the whole microbead. The term "IGF-1 coated microbeads" or "microbeads coated with IGF-1" refers to microbeads that were incubated in a solution containing IGF-1 after bead fabrication. IGF-1 is mainly attached to the bead surface after incubation.

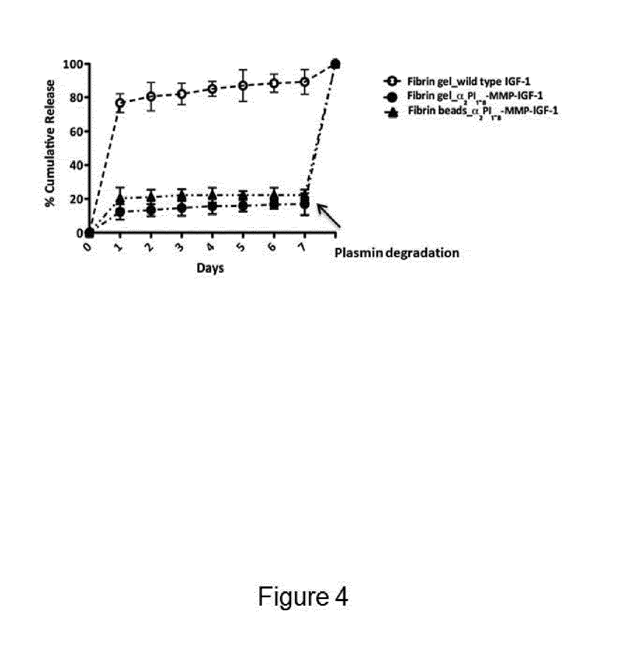

Figure 4 shows cumulative release profiles of α₂PI₁₋₈-MMP-IGF-1 and wild type IGF-1 from fibrin gels and fibrin beads. Growth factors were conjugated to fibrin gels and beads during production. Over 7 days, the quantity of IGF-1 present in the collected supernatant was determined by sandwich ELISA. On day 7, the fibrin gels and the fibrin beads were degraded using plasmin. The amount of IGF-1 retained within the constructs was determined using ELISA. (MMP: matrix metalloproteinase sensitive cleavage site)

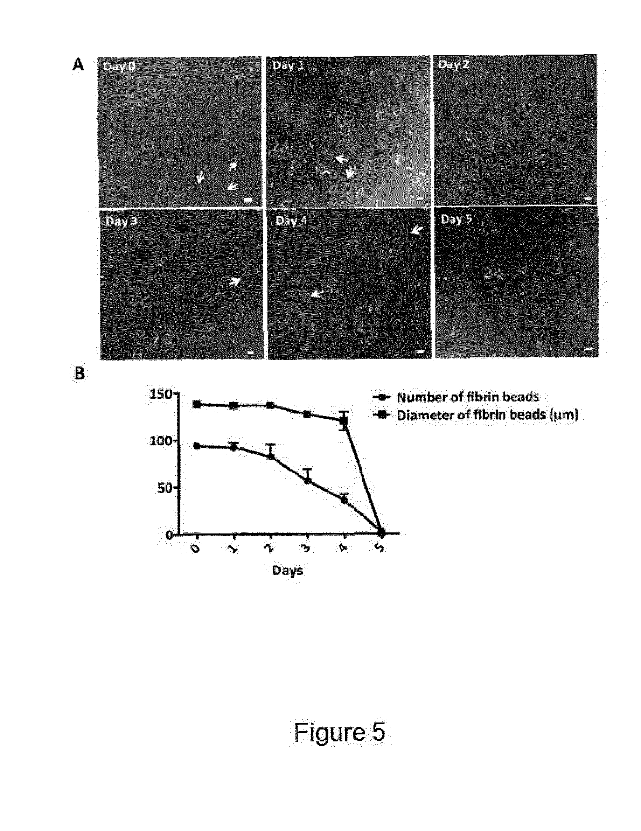

Figure 5 shows in vitro degradation of fibrin beads in the presence of human smooth muscle cells (hSMCs). (A) Bright-field images of fibrin beads in culture with hSMCs from day 0 to day 5. Arrows show the swollen beads. (B) Number and diameter of fibrin beads at each time point were quantified using exported bright field images and processing them with the software ImageJ. Scale bars represent 100 µm.

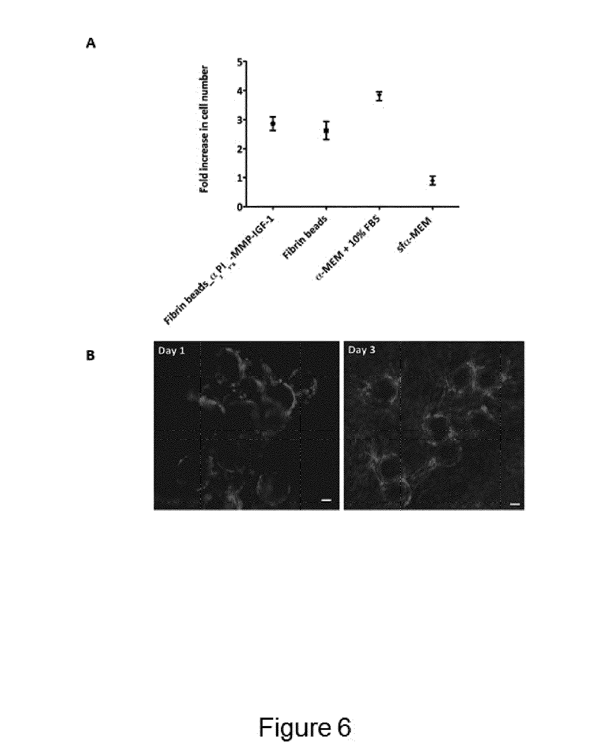

Figure 6 shows proliferation and viability of hSMCs cultured in the presence of fibrin beads. (A) hSMCs were incubated with fibrin beads either conjugated with α₂PI₁₋₈-MMP-IGF-1 or no growth factor. AlamarBlue-specific fluorescence was measured on day 0 and day 3 and the fold increase in cell number was calculated using an established standard curve. Error bars represent the standard deviation of 4 independent samples. (B) Live/Dead staining was performed on hSMCs growing in the presence of fibrin beads conjugated with α₂PI₁₋₈-MMP-IGF-1. Stained samples were visualized under a fluorescent microscope on day 1 and day 3 after bead addition to the cell culture. Scale bars represent 50 µm.

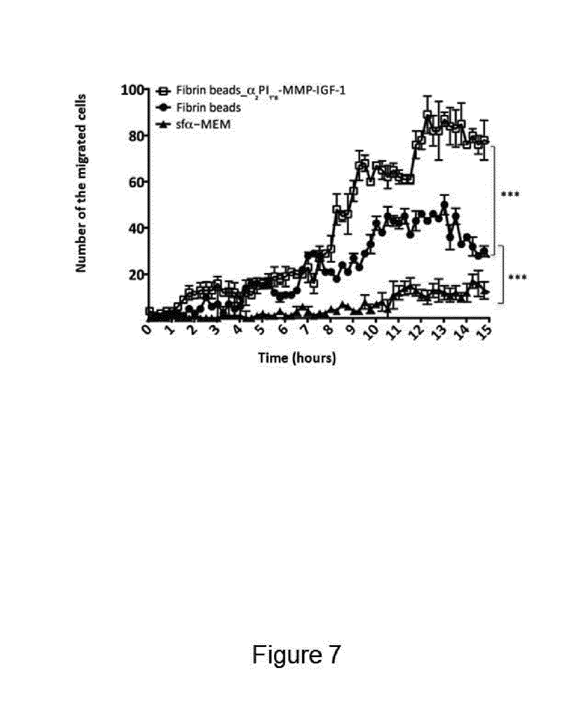

Figure 7 shows migration of hSMCs cultured in the presence of fibrin beads. A transwell migration assay was performed using GFP-expressing hSMCs. The bottom side of the well was filled with either serum free (sf) α-MEM or sf α-MEM supplemented with α₂PI₁₋₈-MMP-IGF-1 conjugated fibrin beads or α-MEM supplemented with 1 % FBS and fibrin beads. The cell migration towards the bottom side of the well was monitored using the Cell IQ imaging system and the data was analyzed using the Cell IQ Analyzer software. (***p<0.001).

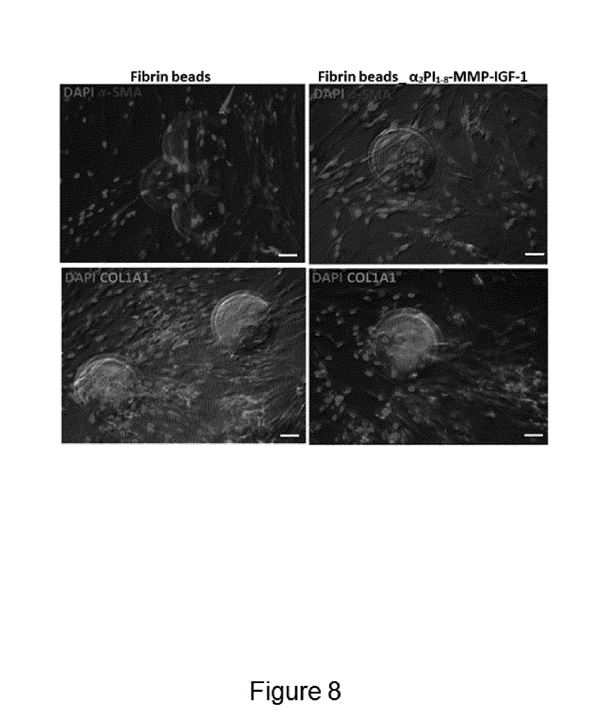

Figure 8 shows smooth muscle alpha actin (α-SMA) and collagen type 1 (COL1A1) expression of hSMCs in the presence of α₂PI₁₋₈-MMP-IGF-1 conjugated fibrin beads. Fixed samples were incubated with anti- α-SMA or anti-COL1A1 antibodies followed by incubation with the corresponding FITC-conjugated secondary antibodies. Cell nuclei were counterstained with DAPI. Stained samples were visualized under a fluorescent microscope. Scale bars represent 50 µm.

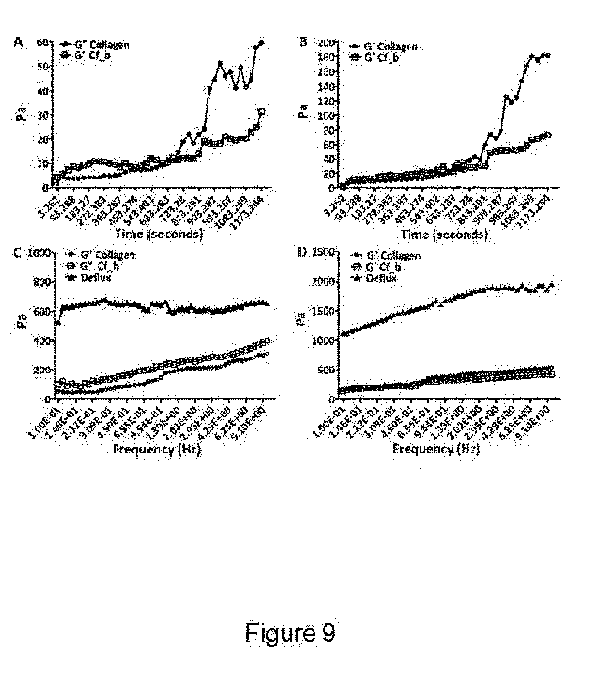

Figure 9 shows rheological properties of collagen gels and fibrin beads embedded within collagen gels (Cf_b). (A and B) Gelation behaviour of the pure collagen solution and collagen solution containing fibrin beads. (A) Loss modulus (G") and (B) storage modulus (G') values of both solutions are plotted in function of time at 37°C. (C) G" and (D) G' values for collagen gels, Cf_b gels, and Deflux. The change in G" and G' values at a frequency range from 0.1 to 10 Hz, was evaluated at room temperature.

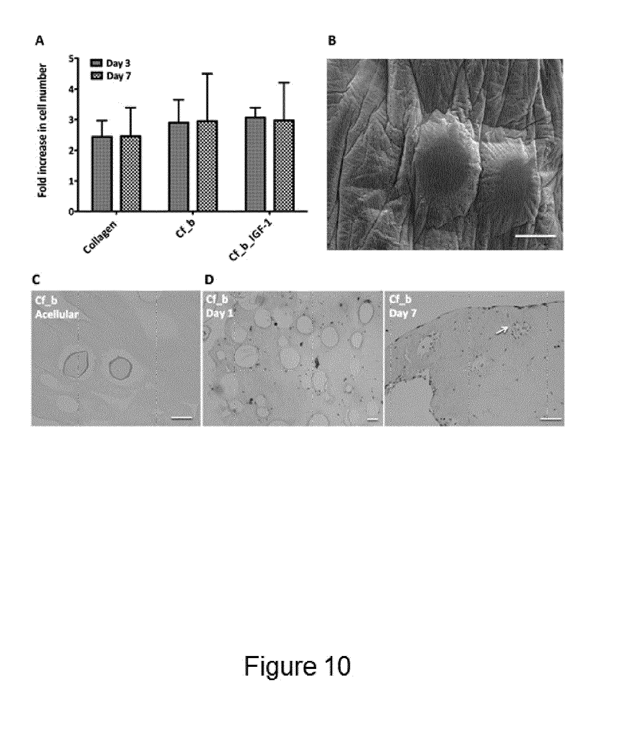

Figure 10 shows hSMCs proliferation and fibrin bead distribution within collagen- gels. Cells were either incorporated within pure collagen gels, collagen gels containing fibrin beads (Cf_b), or collagen gels containing IGF-1 conjugated fibrin beads (Cf_b_)GF-1). (A) AlamarBlue-specific fluorescence was determined at day 3 and day 7 and transferred into fold increase in cell number using an established standard curve. Error bars represent the standard deviation of 4 independent samples. (B) Scanning electron microscopy (SEM) image and (C) Hematoxylin and eosin (HE) stained sample of acellular Cf_b constructs. (D) HE staining of cellular Cf_b gels, showing hSMCs and fibrin beads distribution 7 days after gel fabrication. The white arrow points towards hSMCs attached to a fibrin bead. Scale bars represent 100 µm.

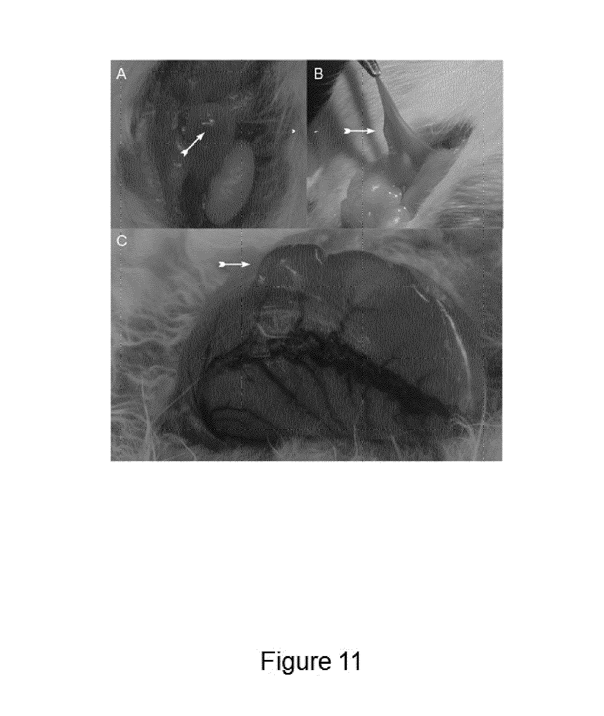

Figure 11 shows injection of ECM based bulk (collagen) in the bladder wall of a rat (A and B) and a rabbit (C). White arrows indicate the bulk created by the injected ECM.

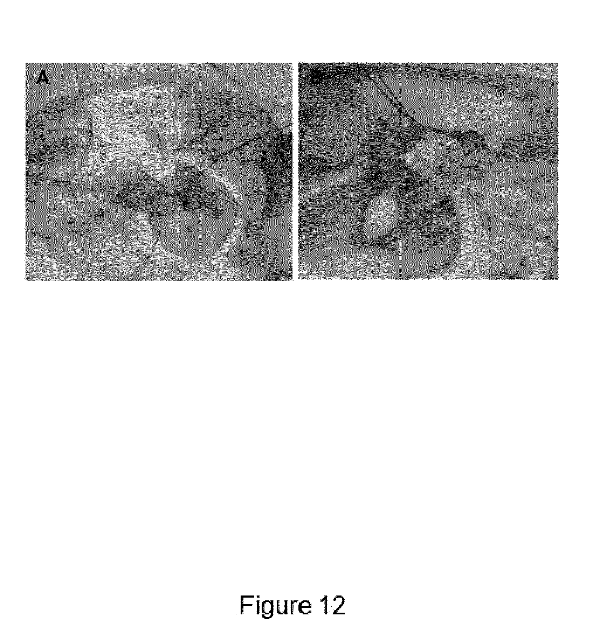

Figure 12 shows a rat bladder with an implanted suturable collagen-fibrin scaffold. The dome of the bladder was excised and the artificially created defect was closed with a collagen-fibrin patch. (A) The scaffold was put into place with the help of holding sutures fixed at the four cardinal points. (B) Collagen-fibrin scaffold is sutured to the rat bladder to close the defect.

### Description of embodiments

The present disclosure may be more readily understood by reference to the following detailed description presented in connection with the accompanying drawing figures, which form a part of this disclosure. It is to be understood that this disclosure is not limited to the specific conditions or parameters described and/or shown herein, and that the terminology used herein is for the purpose of describing particular embodiments by way of example only and is not intended to be limiting of the claimed disclosure.

As used herein and in the appended claims, the singular forms "a", "an" and "the" include plural referents unless the context clearly dictates otherwise. Thus, for example, reference to "a bead" includes a plurality of such beads and reference to "a bioactive agent" includes reference to one or more agents, and so forth.

Also, the use of "or" means "and/or" unless stated otherwise. Similarly, "comprise," "comprises," "comprising", "include," "includes," and "including" are interchangeable and not intended to be limiting. It is to be further understood that where descriptions of various embodiments use the term "comprising", those skilled in the art would understand that in some specific instances, an embodiment can be alternatively described using language "consisting essentially of" or "consisting of." The invention will be better understood with the help of the following definitions.

A main object of the present invention relates to a scaffold material for use in tissue engineering, characterized in that it comprises a plurality of polymeric microbeads embedded within a polymeric carrier, wherein the microbeads and the carrier are substantially composed of the same or different natural polymeric material or extracellular matrix-derived polymeric material. The invention presented herein is based at least in part on the development of a novel manufacturing method for producing micro-sized beads substantially composed of a polymeric material, preferably but not exclusively natural polymeric material such as polymers derived from the extracellular matrix (hereinafter also referred to as "ECM"). As used herein, a "microbead" or "micro-bead" refers to micro-sized particles. Such manufacturing method facilitates the production of the scaffold materials of the invention and optimize its short-term bulking effect coupled with its long term functional effect in both *in vivo* and *in vitro* applications. In fact, as will be detailed below, the manufacture of the microbeads can be finely tuned and tailored according to the needs of an operator in order to achieve a perfect balance between structure and function of the scaffold, particularly when microbeads include an active agent for tissue functional recovery.

In the frame of the present disclosure, a "scaffold material" is any three dimensional material having a framework architecture, i.e. a support structure comprising hollow spaces within it. Generally speaking, a scaffold material is an artificial structure capable of supporting three-dimensional body tissue/organ formation *in vivo, ex vivo* or *in vitro.* In this context, a scaffold material is also referred herewith as a "biomaterial" or "bioscaffold". A bioscaffold, *inter alia,* allows cell attachment and migration, delivers and retains cells and biochemical factors, enables diffusion of vital cell nutrients and expressed products, exerts certain mechanical and biological influences to modify the behaviour of the cell phase and so forth.

The scaffold material of the invention has been conceived and manufactured in order to act as a biocompatible, non-migratory, non-carcinogenic and non-immunogenic bulking agent. The general purpose was the development of a bulking formulation having improved long-term efficacy, which can stimulate host cell infiltration and integrates with the surrounding tissue once implanted in a host, thus triggering neo-tissue formation via the promotion of a bioactive environment within the application (e.g. injection) site and giving rise to long-term functional repair. The scaffold material herein disclosed addresses and solves, among others, this problem.

As used herein, a "polymeric material" is any material comprising polymers, large molecules (also known as macromolecules) composed of many repeated smaller units, or subunits, called monomers, tightly bonded together preferably by covalent bonds. Polymer architecture at the molecular scale can be rather diverse. A linear polymer consists of a long linear chain of monomers. A branched polymer comprises a long backbone chain with several short side-chain branches covalently attached. Cross-linked polymers have monomers of one long or short chain covalently bonded with monomers of another short or long chain. Cross-linking results in a three-dimensional molecular network; the whole polymer is a giant macromolecule. Another useful classification of polymers is based on the chemical type of the monomers: homopolymers consist of monomers of the same type, copolymers have different repeating units. Furthermore, depending on the arrangement of the types of monomers in the polymer chain, there are the following classification: the different repeating units are distributed randomly (random copolymer) or there are alternating sequences of the different monomers (alternating copolymers) in block copolymers long sequences of one monomer type are followed by long sequences of another type; and graft copolymers consist of a chain made from one type of monomer with branches of another type. A sufficiently dense polymer solution can be crosslinked to form a polymer gel, including a hydrogel or a cryogel, which is a soft solid.

Polymer materials may also be formed by blending two or more polymers into physical mixtures. For example, the rather poor impact strength of polystyrene is greatly improved by incorporating small particles of an elastomer. Many properties of polymeric materials depend on the microscopic arrangement of their molecules. Polymers can have an amorphous (disordered) or semicrystalline (partially crystalline, partially ordered) structure. Polymers can be mixed with inorganic particles (usually in the form of continuous fibres, such as glass or particulates such as mica, talc and clay) in order to modify and improve (mainly but not exclusively) their mechanical properties.

As used herein, a "carrier" is any substance which function as a dispersing means for the microbeads of the invention and which allows a suitable delivery means for these latter. In preferred embodiments of the invention, the carrier is a soft carrier material, i.e. it is compressible, preferably reversibly compressible, malleable, ductile and/or plastic, and can comprise or consist of a polymeric matrix, i.e. and organised or amorphous network of monomeric elements. Said polymeric matrix may comprise one or more compounds selected from a non-exhaustive list comprising natural polymeric material (i.e., non-synthetic polymers, polymers that can be found in nature) and/or polymers derived from ECM as gelatin, elastin, collagen, agar/agarose, chitosan, fibrin, proteoglycans, a polyamino-acid or its derivatives, preferably polylysin or gelatin methyl cellulose, carbomethyl cellulose, polysaccharides and their derivatives, preferably glycosaminoglycanes such as hyaluronic acid, chondroitinsulfate, dermatansulfate, heparansulfate, heparine, keratansulfate or alginate, nucleotides, polylipides, fatty acids, as well as any derivative thereof, fragment thereof and any combination thereof.

The carrier of the scaffold material of the present invention is provided in the form of a bulk, a paste, a gel or a hydrogel. Therefore, the scaffold material of the invention may be in a variety of forms, the preferred one depending on the intended mode of administration and therapeutic application. Typically preferred compositions are in the form of needle-injectable hydrogels, but semi-solid or putty-like formulations can also be envisaged. For instance, a polymeric gel in lyophilized form as bulk material ("plug") can be placed at the target body site and it can swells in vivo once in contact with body fluids.

As used herein, the term "gel" refers to a non-fluid colloidal network or polymer network that is expanded throughout its whole volume by a fluid. A gel is a solid three-dimensional network that spans the volume of a liquid medium and ensnares it through surface tension effects. The internal network structure may result from physical bonds (physical gels) or chemical bonds (chemical gels).

As used herein, the term "hydrogel" refers to a gel in which the swelling agent is water. A hydrogel is a macromolecular polymer gel constructed of a network of crosslinked polymer chains. It is synthesized from hydrophilic monomers, sometimes found as a colloidal gel in which water is the dispersion medium. Hydrogels are highly absorbent (they can contain over 90% water) natural or synthetic polymeric networks. As a result of their characteristics, hydrogels develop typical firm yet elastic mechanical properties.

Several physical properties of the (hydro)gels are dependent upon concentration. Increase in (hydro)gel concentration may change its pore radius, morphology, or its permeability to different molecular weight proteins. One skilled in the art will appreciate that the volume or dimensions (length, width, and thickness) of a (hydro)gel can be selected based on instant needs, such as for instance the region or environment into which the (hydro)gel is to be implanted. Particularly in a hydrogel formulation (that is, where the carrier is in the form of a hydrogel), the scaffold material of the invention is characterized by its ability to act as an efficient bulking agent, which make it an ideal delivery vehicle for bioactive agents embedded into microbeads to a target region in a subject, especially for soft tissue engineering applications.

The mechanical properties of the material can be tailored according to said needs by changing the physical or chemical properties thereof (molecular chain length, crosslinking rate, water content, and so forth). In this context, in order to optimize the mechanical properties of the scaffold material of the invention and, in some aspects, its resorption/biodegradation rate, in preferred embodiments it is contemplated an average molecular weight for the macromolecules substantially composing the polymeric material of the carrier comprised between about 50 and 600 kDa, and a polymer density of at least 1 mg/mL. A polymer density comprised between 2 and 5 mg/mL is considered to be a suitable density for the carrier material according to the invention. In most preferred embodiments, the polymeric carrier material is not crosslinked or minimally crosslinked in order to keep the bioscaffold in a suitable needle-injectable form. Crosslinking agents and their amount can be chosen at the operator's discretion, and a skilled in the art would easily envisage such parameters based on common practice.

Concerning the degradation/resorption rate of the carrier, particularly upon in vivo application/implant in a host, this is mainly dependent on phisico-chemical properties of the polymeric material of which it is composed of, as well as further factors such as crosslinking of the polymers, the polymer concentration, the site of implant into a host and the like. Generally speaking, for both the carrier and the microbeads, the polymers may be intrinsically biodegradable *in vivo,* but they are preferably chosen of a low biodegradability rate (for predictability of dissolution).

In general terms, the carrier portion constitutes at least the 1 % of the volume of the entire scaffold. However, due to its nature and purpose, the carrier of the present invention may compose a remarkable fraction of the scaffold material of the invention, both in terms of volume and mass, so that as a final result the entire scaffold can have substantially the mechanical properties of said carrier. For example, when a soft carrier is used for producing the scaffold material, this latter can result soft too. In certain embodiments, the carrier portion can advantageously constitute between 40 to 80% of the total scaffold volume. The inclusion of microbeads within the carrier does not generally alter in a considerable way the mechanical properties of the scaffold (e.g., the viscoelasticity), particularly when the microbeads are evenly embedded in the carrier. For example, the viscous modulus G" and the elastic modulus G' can be substantially indistinguishable between the carrier and the scaffold material of the invention comprising the same carrier with embedded microbeads. The number of the microbeads is chosen depending on the needs (type of organ/tissue, application means, final functional response to be achieved and so forth), as will be detailed later on.

The term "microbeads" is used herein to refer to round particles i.e. particles that are substantially spherical and/or substantially ellipsoidal in shape. These structures are dense, compact polymeric structures that do not present any cavity in their core, i.e. they are not hollow core-shell structures. The beads preferably have a mean particle size comprised between 10 and 1000µm, preferably between 80 and 500µm, more preferably between 100 and 200µm. In a most preferred embodiment, particles have a mean particle size of around 150µm. Particle size refers to the length of the longest dimension of the particles. Combination of microbeads having a different size and shape can be envisaged. The size of the beads can be chosen depending on the instant needs (e.g., degradation rate, active agents embedded therein and the like), but they are preferably sized in order to behave as non-migratory particles in the injection site over time in an *in vivo* setting; this aim is reflected in the preferred embodiments concerning the beads' size as disclosed above.

A skilled person will appreciate that the present disclosure is meant to include structures in a nanometric scale rather than in a micrometric scale. The beads' size can be tailored based on specific needs by, e.g., altering the condition for producing them, as will be detailed later on. However, for most of the applications of the beads of the invention, a micrometric scale is considered to be the best option.

As for the carrier portion of the scaffold material according to the invention, some examples of suitable constituents of the polymeric microbeads of the invention include, but are not limited to, natural polymeric material and/or polymers derived from ECM as gelatin, elastin, collagen, agar/agarose, chitosan, fibrin, proteoglycans, a polyamino-acid or its derivatives, preferably polylysin or gelatin methyl cellulose, carbomethyl cellulose, polysaccharides and their derivatives, preferably glycosaminoglycanes such as hyaluronic acid, chondroitinsulfate, dermatansulfate, heparansulfate, heparine, keratansulfate or alginate, nucleotides, polylipides, fatty acids, as well as any derivative thereof, fragment thereof and any combination thereof. Both for the carrier and the microbeads, natural and ECM derived polymers are a first choice biomaterial for tissue engineering applications envisaged by the present disclosure, due to their biological and chemical similarities to natural tissues and the presence of biologically active sites in their structures. For example, fibrin can be considered as the most preferred embodiment for constituting the microbeads of the invention, due to its inherent integrin and growth factor-binding sites, and its controllable, natural protease-dependent degradation in vivo.

As for the carrier, in order to optimize some of the properties of microbeads (e.g. mechanical properties or the degradation rate), in preferred embodiments it is contemplated an average molecular weight for the macromolecules substantially composing the polymeric material of the microbeads comprised preferably between about 50 to about 600 kDa, and a polymer density comprised at least 1 mg/mL, with the most preferred density being comprised between 20 and 30 mg/mL. The microbeads amount is chosen depending on several factors as already outlined, and the total microbeads volume can span between 1 to 99 % of the total volume of the scaffold material, preferably between 20 to 60 %. Moreover, in one embodiment, the ratio between the microbeads polymer density and the carrier polymer density is 2.2/4.5.

The degradation rate of the microbeads can be calibrated by adjusting certain physico-chemical parameters thereof, such as for instance by polymer crosslinking, the use of inhibitor molecules, by increasing the polymer density, crystallinity and/or its molecular weight distribution, changing the beads' porosity and so forth.

For instance, since large pore sizes create favourable conditions for enzymes to penetrate into a material, in preferred embodiments of the invention the microbeads are substantially smooth on their surface, with a high surface porosity and very small pore sizes (between 50nm and 400nm). This is particularly useful for slowing down the (*in vivo*) degradation of the beads as well as for delaying the release of an active agent, if any, embedded therein. In this regard, in some embodiments, the *in vitro* degradation rate of polymeric (e.g. fibrin) microbeads incubated with a suitable number of cells is considerably slower than equivalent polymeric (e.g. fibrin) gels.

Cells are continuously secreting proteases that result in polymer degradation; thus, the degradation products of cells can initially diffuse easily into both macro- and micro- size structures. However, these enzymes diffuse out of the macro structures slower than the smaller micro particles, leading to faster degradation. In this context, almost no protein release from the polymeric microbeads can be observed two days after incubation in an *in vitro* setting.

Moreover, always in an *in vitro* setting, it has been found that at least some of the beads swell if put in culture with cells. In some embodiments, microbeads of the invention can increase their size about 1.5 times compared to their original diameter. The swelling of the microbeads might be part of their polymer degradation: when a polymer has a compact surface, the degradation products of this polymer cannot easily exit from the inside, and this accumulation of degradation products inside polymer chains might lead to swelling of the polymer so that the beads might be swollen before they undergo surface degradation. This favours not only the delay in the degradation of the polymeric microbeads, but also the controlled, long term release of active agents embedded therein.

In preferred embodiments of the invention, the microbeads are characterized in that they comprise bioactive molecules. Said bioactive molecules can be coated or otherwise attached to the surface of the beads, but in the most preferred embodiment they are embedded within the beads, most preferably in a homogeneous manner. A homogenous distribution of bioactive molecules within microbeads, *inter alia,* increases the range of compounds' dosage that can be loaded into a bead (i.e. coupling bioactive molecules to the microbeads' surface is more limited due to the available surface), protects more efficiently the bioactive molecules and prolongs/regulates the release of the bioactive molecules, particularly when used in a tissue engineering scenario.

In the frame of the present disclosure, the expression "bioactive molecule", as well as "bioactive compound", "active agent", "bioactive agent" or "therapeutic agent", refers to any agent that is biologically active, i.e. having an effect upon a living organism, tissue, or cell. The expression is used herein to refer to a compound or entity that alters, inhibits, activates, or otherwise affects biological or chemical events. Bioactive compounds according to the present disclosure can be small molecules or preferably macromolecules, including recombinant ones.

One skilled in the art will appreciate that a variety of bioactive compounds can be used depending upon the needs, e.g. a condition to be treated when the microbeads of the invention are intended for prophylactic or therapeutic uses such as for tissue engineering. Exemplary therapeutic agents include, but are not limited to, a growth factor, a protein, a peptide, an enzyme, an antibody or any derivative thereof (such as e.g. multivalent antibodies, multispecific antibodies, scFvs, bivalent or trivalent scFvs, triabodies, minibodies, nanobodies, diabodies etc.), an antigen, a nucleic acid sequence (e.g., DNA or RNA), a hormone, an anti-inflammatory agent, an anti-viral agent, an anti-bacterial agent, a cytokine, an oncogene, a tumor suppressor, a transmembrane receptor, a protein receptor, a serum protein, an adhesion molecule, a lypidic molecule, a neurotransmitter, a morphogenetic protein, a differentiation factor, an analgesic, organic molecules, metal particles, radioactive agents, polysaccharides, a matrix protein, a cell, and any functional fragment or derivative of the above, as well as any combinations thereof. For "functional fragment" is herein meant any portion of an active agent able to exert its physiological/pharmacological activity. For example, a functional fragment of an antibody could be an Fc region, an Fv region, a Fab/F(ab')/F(ab')₂ region and so forth.

For "derivative" is herein meant a compound that is derived from a similar compound by some chemical or physical process. Fusion proteins or poly/oligopeptides, metal-coupled macromolecules, radioactive agents-coupled macromolecules and the like are non-limiting examples of compound derivatives. A derivative can be chosen or created on the basis of the instant needs, for instance for delaying the release of an active agent from the core of a microbead of the invention. As a way of example, in one embodiment an oligopeptide-growth factor fusion protein can be embedded within the beads in order to stabilize the internal structure of the microbeads, slow down the release of the active agent, diminish the degradation rate of the microbeads, favour the migration and/or invasion-infiltration of endogenous cells within the scaffold material upon implantation in a host and so forth.

The scaffold material can be delivered or applied to a specific region in a host. In the frame of the present disclosure, the term "host" can be used interchangeably with the term "subject", unless otherwise stated. Thus, in a particular aspect, the invention pertains to the localized delivery or application of the scaffold material of the invention to a target body region in a subject.

In a preferred embodiment, the scaffold material is formulated into a flowable and needle-injectable form. If required, the scaffold material can be mixed with an amount of water or physiologically compatible buffer sufficient to produce the desired consistency for injection. Most often this will be studied for being able to pass through a 16, 18, 20, 24 or 26 gauge syringe needle. Other gauged syringes may also be used such as a 12-14 gauge syringe, as well as larger structures such as catheters, cannulas or larger dosing tips when applying the material to e.g. superficial tissue surfaces. For example, a scaffold material provided as a hydrogel formulation (e.g., wherein the carrier is a hydrogel) is a first choice option for a needle-injectable formulation. For some formulations requiring injection directly into solid tissue (into e.g. cancellous bone of an osteoporosis patient), thinner consistencies may be used. In a preferred embodiment, the mode of administration is through in situ injection (i.e., injection of the composition directly in the area to be treated). The methods and scaffold material of the invention can thus also provide a minimally invasive technique for the treatment of difficult clinical situations.

Thanks to the presence of the above-described bioactive molecules-loaded microbeads, the scaffold material releases one or more active agents at the target region of the subject in a controlled manner and eliminates the drawbacks concerning e.g. a fast or otherwise barely tuneable release of a therapeutic agent upon local administration. In particular, the use of the scaffold material of the invention can avoid, as already described above, a "burst release" of the therapeutic agent and promotes, especially for soft tissue engineering applications, a short-term bulking effect while providing a long-term functional repair without causing inflammation and scar tissue formation. In fact in a first approximation, in view of all the features of the microbeads of the invention, the bioactive molecules embedded within said microbeads are released upon degradation of the latter in a substantially linear fashion.

The scaffold material of the invention can further comprise at least one additional therapeutic agent, e.g. antibiotic, growth factors or one or more additional therapeutic agents for treating a condition in which use of the scaffold material is beneficial to amelioration of said condition.

The scaffold material described herein is useful in the treatment of a variety of diseases, disorders, and defects where a tissue engineering approach can be a suitable therapeutic solution. In this context, the scaffold material of the invention can be an excellent solution as a bulking agent in order to increase the bulk volume while improving e.g. the coaptation of a damaged tissue such as a mucosa: it results mechanically stable and non-immunogenic, mainly thanks to the natural polymeric materials of choice, and provides at the same time a long-term functional restoration of the treated tissue/organ. This is particularly true for soft tissues, and the scaffold material may therefore be utilized in a variety of surgical procedures as well as for cosmetic purposes, and for the treatment or prevention of a plethora of pathological conditions. The scaffold material of the invention results particularly convenient for treating tissues or organs like a urinary tract component (including kidney), a blood vessel (including big veins and arteries), a muscle, a cartilage, skin, liver, a cornea, trachea, esophagus, heart, pharynx or inner ear tissue.

The scaffold material can moreover be filled in cavities present in non-biodegradable body implants or surgical tools or applied to the surface of those devices. An application of the material according to the present invention through implants presenting channels or grooves such as cannulated screws can be imagined as well. Furthermore, the material can be embedded in existing biodegradable implants like resorbable screws or plates, or added to existing formulations like biodegradable bone cements, rinsing solutions or implant coatings.

As used herein, "treatment", "treating" and the like generally means obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in an animal, preferably a mammal, particularly a human, and includes: (a) preventing the disease from occurring in a subject which may be predisposed to the disease but has not yet been diagnosed as having it for example based on familial history, overweight status or age; (b) inhibiting the disease, i.e., arresting its development; or relieving the disease, i.e., causing regression of the disease and/or its symptoms or conditions such as improvement or remediation of damage.

The term "subject" as used herein refers to animals, particularly mammals. For example, mammals contemplated by the present invention include human, primates, domesticated animals such as cattle, sheep, pigs, horses, laboratory rodents and the like.

As will be evident for a skilled person, the amount of the bioactive agent(s) present within the scaffold material (i.e. preferably embedded within the microbeads) is selected to be a therapeutically effective amount. The expression "therapeutically effective amount" as used herein means that amount of a compound (e.g. a material, (macro)molecule or composition) which is effective for producing some desired therapeutic effect in a subject at a reasonable benefit/risk ratio applicable to any medical treatment. Accordingly, a therapeutically effective amount may, for example, prevent, minimize, or reverse disease progression associated with a disease or bodily condition. Disease progression can be monitored by clinical observations, laboratory and imaging investigations apparent to a person skilled in the art. A therapeutically effective amount can be an amount that is effective in a single dose or an amount that is effective as part of a multi-dose therapy, for example an amount that is administered in two or more doses or an amount that is administered chronically.

The effective amounts will depend upon a variety of factors such as the severity of the condition being treated; individual patient parameters including age, physical condition, sex, size and weight; concurrent treatments; the frequency and/or duration of treatment; general health and prior medical history of the patient being treated, and like factors well known in the medical arts.

The term "efficacy" of a treatment or method according to the invention can be measured based on changes in the course of disease or condition in response to a use or a method according to the invention. For example, the efficacy of a treatment or method according to the invention can be measured by clinical relief of above-mentioned somatic symptoms.

The invention also provides pharmaceutical compositions comprising the scaffold material of the invention. These compositions may, optionally and additionally, comprise a pharmaceutically acceptable carrier, excipient and/or diluent. As used herein, "pharmaceutically acceptable carrier" includes any and all solvents, dispersion media, coatings, antibacterial and antifungal agents, isotonic and absorption delaying agents and the like, that are physiologically compatible. Examples of suitable pharmaceutical carriers are well known in the art and include sodium chloride solutions, phosphate buffered sodium chloride solutions, water, emulsions, such as oil/water emulsions, various types of wetting agents, sterile solutions, organic solvents and so forth. The pharmaceutically acceptable carrier suitably contains minor amounts of additives such as substances that enhance isotonicity and chemical stability. Such materials are non-toxic to recipients at the dosages and concentrations employed, and include buffers such as e.g. phosphate, citrate, succinate, acetic acid, and other organic acids or their salts; antioxidants such as ascorbic acid; low molecular weight (less than about ten residues) (poly)peptides, e.g., polyarginine or tripeptides; proteins such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The scaffold material of the present invention can benefit, in preferred embodiments, of a new method for manufacturing the microbeads through the microfluidic technology, particularly the application concerning the use of a microfluidic chip for generation of droplets. The method conceived by the inventors provides a fast, reliable and robust setup for obtaining natural polymer or ECM-derived polymer microbeads of fine-tunable and optimized characteristics for the purposes of the invention.

In the frame of the present disclosure, a "microfluidic device", "microfluidic chip" or "microfluidic platform" is any apparatus, which is conceived to work with fluids at a micro/nanometer scale. Microfluidics is the science that deals with the flow of liquid inside channels of micrometer size. At least one dimension of the channel is of the order of a micrometer or tens of micrometers in order to consider it microfluidics. Microfluidics can be considered both as a science (study of the behaviour of fluids in micro-channels) and a technology (manufacturing of microfluidics devices for applications such as lab-on-a-chip). These technologies are based on the manipulation of liquid flow through microfabricated channels. Actuation of liquid flow is implemented either by external pressure sources, external mechanical pumps, integrated mechanical micropumps, or by combinations of capillary forces and electrokinetic mechanisms.

The microfluidic technology has found many applications such as in medicine with the laboratories on a chip because they allow the integration of many medical tests on a single chip, in cell biology research because the micro-channels have the same characteristic size as cells and allow amongst others the manipulation of single cells and rapid change of drugs, in protein crystallization because microfluidic devices allow the generation of a large number of crystallization conditions (i.e. temperature, pH, humidity) on a single chip and also in many other areas such as drug screening, sugar testers, chemical micro reactors, or micro fuel cells.

Generally speaking, a microfluidic chip is a set of micro-channels etched or molded into a material (glass, silicon or polymers such as PDMS). The micro-channels forming the microfluidic chip are connected together in order to achieve a desired function (mix, pump, redirect and/or allow chemical reactions in a cell). This network of micro-channels trapped in the microfluidic chip is connected to the outside by inputs and outputs pierced through the chip, as an interface between the macro- and micro-world. It is through these holes that fluids (either liquids, gases or combinations thereof) are injected and removed from the microfluidic chip (through tubing, syringe adapters or even free holes in the chip).

The simplest microfluidic devices consist in micro-channels molded in a polymer that is bonded to a flat surface (a glass slide as an example).The polymer most commonly used for molding microfluidic chips is polydimethylsiloxane (PDMS). The PDMS is a transparent, biocompatible (very similar to silicone gel used in breast implants), deformable, inexpensive elastomer, easy to mold and bond with glass.

The manufacture of a microfluidic device starts with the design of the channels on a dedicated software. Once this design is made, it is sent to a manufacturer of photomask to be transferred on a glass medium or a plastic film. The micro-channels are usually printed with UV opaque ink (if the medium is a plastic film) or chromium (if the medium is a glass plate). Then, the drawings of the microchannels on the photomask are transformed into real micro-channels (the mold). Negative micro-channels are "sculpted" on the mold, resulting in replicas that will enable the carving of the channels into the future material of the microfluidic chip.

In some embodiments, the microfluidic chip according to the invention comprises or consists of:
- at least two sample reservoirs operatively connected with a pressure source adapted to apply a positive pressure thereon, at least one of said reservoirs being intended for containing an aqueous solution comprising a precursor of the polymeric material substantially composing the microbeads and at least one of said reservoirs being intended for containing an aqueous solution comprising a polymerization catalyser;
- at least one channel operatively connected with each of the said sample reservoirs through their inlets;
- a mixing point operatively connecting the outlets of each of said channels;
- at least one microsized channel stemming from said mixing point;
- at least one organic phase reservoir operatively connected with a pressure source adapted to apply a positive pressure thereon, said reservoir being intended for containing an organic solution;
- at least one microsized channel operatively connected with each of the said organic phase reservoirs through its inlet, this microsized channel intersecting the microsized channel stemming from the mixing point in a beads-forming point;
- a focusing element stemming from said beads-forming point adapted so to canalize the microbeads; and
- a microbeads reservoir operatively connected with both the focusing element and means for regulating the temperature in the reservoir.

As used herein, the wording "operatively connected" reflects a functional relationship between two or more components of a device or a system, that is, such a wording means the claimed components must be connected in a way to perform a designated function. The "designated function" can change depending on the different components involved in the connection; for instance, a pressure source operatively connected to a reservoir has the function to alter the reservoir's internal pressure in a positive or negative fashion; in the same way, a microchannel operatively connected with a reservoir must be such that the content of said reservoir must be able to flow throughout the said microchannel.

The microfluidics chip according to the present disclosure is conceived to work as a two-phase flow microfluidics chip, a technology platform developed, among others, for the formation and/or merging of droplets inside an immiscible carrier fluid. Two-phase microfluidic flows are generated when two partially miscible or immiscible fluids are brought into contact in microfluidic devices.

Many different techniques have been developed to obtain fine control over the size and shape of droplets. Techniques for producing droplets can be either passive or active, the latter meaning that external fields are activated at the time and on-chip location where droplets need to be formed. The majority of techniques are passive and produce a continuous stream of evenly spaced drops. In this scenario, the flow field causes the interface between the two fluids to deform, leading to a growth of interfacial instabilities.

In general, the fluid phase to be dispersed is brought into a microchannel by a pressure-driven flow, while the flow of the second immiscible carrier liquid is driven independently. These two phases meet at a junction, where the local flow field, determined by the geometry of the junction and the flow rates of the two fluids, deforms the interface. Eventually droplets pinch off from the dispersed phase finger by a free surface instability. The pinch-off of droplets is largely dictated by the competition between viscous shear stresses acting to deform the liquid interface and capillary pressure acting to resist the deformation.

The three most common strategies for obtaining droplets in a microfluidics setting are the use of T-junction, Y-junction or flow focusing geometries. In a typical T-junction configuration, the two phases meet face to face and then flow through orthogonal channels, forming droplets where they meet. A Y-junction configuration is a modification of the T-junction setting wherein the two feeding microchannels (one for the continue phase and one for the dispersed phase) meet with a relative inclination angle different from 0°.

In the flow focusing technique, a continue phase fluid exerts pressure and tangential viscous stress over a dispersed phase fluid, so to force this latter into a microthread that breaks up in the vicinity of an orifice through which both fluids are extruded through capillary instability. All the above described microfluidic chip configurations for obtaining micro/nanodroplets are well known techniques readily available to a skilled person, and a complete review thereof can be found in Gu et al. (Int. J. Mol. Sci. 2011, 12, 2572-2597).

The device comprises a means to directly or indirectly alter the pressure within the reservoirs, i.e. any kind of suitable pressure source. The pressure applied can be a "positive pressure", i.e. when the applied pressure increases the internal reservoir fluid pressure, or a "negative pressure", i.e. when the applied pressure diminishes the internal reservoir fluid pressure, as in case of a suction. A means to apply a pressure is coupled with a reservoir either directly or indirectly (via e.g. a connection tube). Suitable means of altering the pressure within the device are external or integrated pumps or micropumps, combinations of capillary forces and electrokinetic mechanisms, hydrostatic pressure or simply a syringe. In some embodiments, each such pressure source is individually addressable so that the reservoirs' content can be, even dynamically, regulated and fine-tuned in order to deliver the needed amount of precursors and/or catalyser. This aspect of the microfluidic chip, i.e. the possibility to regulate the pressure inside each single reservoir, is particularly useful and advantageous for tailoring many of the physico-chemical parameters of the microbeads. By regulating the amount of precursors, catalysers, bioactive molecules and/or organic phase (detailed later on) released in the chip system, the exact size and composition of possibly each microbead can be specifically controlled and adjusted depending on the needs at each time point.

For the production of the microbeads, precursors of the polymeric material are provided into a "precursor reservoir". Such reservoir can ideally be only one but in some embodiments of the invention there can be more than one. The precursor(s) are, generally speaking, the monomers forming the polymeric material, normally in an aqueous solution. An "aqueous solution" is a solution in which the solvent is substantially made of water. In the frame of the present disclosure, the term "aqueous" means pertaining to, related to, similar to, or dissolved in water.

In a preferred embodiment of the invention, such precursors are precursors of natural polymeric material and/or polymers derived from ECM as sugars, polysaccharides, peptides or polypeptides, either glycosylated or not, such as for instance collagen, fibrinogen, tropoelastin, chitin and the like, fragments thereof, derivatives thereof as well as combinations thereof.

At least one other reservoir of the chip is intended for containing an aqueous solution comprising a polymerization catalyser, hereinafter also referred to as "catalyser reservoir". Such a catalyser starts and promotes at least the first part of the process of polymerization of the polymeric precursor(s), which takes place all along the chip up to the final beads collecting reservoir, and that begins in a junction point of the chip where the precursor(s) and the catalyser are allowed to mix. The catalyser can be a chemical or an aqueous solution comprising it, either acid or basic solution, such as an e.g. sodium hydroxide solution, or can be (an aqueous solution comprising) a macromolecule such as an enzyme, as for instance Lysyl Oxidase, Thrombin, Factor XIII and so forth, or combinations thereof. In a particular embodiment of the invention, fibrin microbeads are produced starting from a fibrinogen precursor and Thrombin + Factor XIII as catalysers. Collectively, the precursor(s) reservoir and the catalyser reservoir are referred to herewith as "the sample reservoirs".

The microfluidic chip comprises a third type of reservoir intended to contain an organic phase (hereinafter also referred to as "organic phase reservoir") operatively connected with a pressure source adapted to apply a positive pressure thereon, and having at least one microsized channel operatively connected thereto through its inlet (hereinafter also referred to as "organic phase microchannel").

As used herein, an "organic phase" is a non-polar solution in which the solvent is a non-polar compound. Non-polar solvents are intended to be compounds having low dielectric constants and that are not miscible with water. Non-polar solutions can comprise for example solutions comprising oils, benzene, carbon tetrachloride, diethyl ether, xylene, toluene, isooctane, ethanol, heptanol, cyclohexane, hexadecane, n-octane and the like. An "oil" is any non-polar chemical substance that is a viscous liquid at ambient temperature and is both hydrophobic and lipophilic. In the frame of the present disclosure, aqueous solutions are also referred to as "water phase" or "polar phase" and non-polar solutions are also referred to as "oil phase".

The microchannel sprouting from the organic phase reservoir is in some embodiments designed and adapted so to create a closed circuit in which the organic phase continuously flows in one direction, in order to exploit always the same amount of it for its applications and assuring a continuous pressurized fluid supply.

The sample reservoirs are operatively connected among them via at least one channel independently stemming from each of them through their inlets and converging to a mixing point operatively connecting the outlets of each of said channels, with any suitable contact angle. Once a positive pressure applied to the precursor reservoir and the catalyser reservoir, the mixing point allows the contact among the contents of those reservoirs and their subsequent merging. The mixing point can be the intersection of the two above-described channels or can even comprise a chamber so that a higher amount of reagents can be brought into contact.

From said mixing point, at least a microsized channel stemming therefrom collects the so obtained pre-polymerized mixture comprising the precursor(s) and the catalyser, and heads towards a junction point with the organic phase microchannel. At this junction point, microbeads of partially polymerized mixture arise by following the above described process of microdroplets formation. The so obtained pre-polymerized microbeads are then canalized through a focusing element stemming from said beads-forming point into a microbeads reservoir operatively connected with both the focusing element and means for regulating the temperature in the reservoir. Said microbeads reservoir is intended for collecting the obtained pre-polymerized microbeads as well as to incubate them for the final, temperature-dependent step of complete polymerization. In this context, the temperature inside the microbeads reservoir can be regulated with any suitable means known in the art up to the necessary level for driving the microbeads polymerization to the end. Moreover, the reservoir can have any suitable shape and dimension in order to accommodate all the produced beads as well as for facilitating the polymerization process; for instance, the microbeads reservoir can consist of or comprise a long microchannel with e.g. a serpentine design in order to protract the polymerization reaction and homogenize the temperature. In a particular embodiment of the invention, fibrin microbeads are produced starting from a fibrinogen precursor and Thrombin + Factor XIII as catalysers, and letting the polymerization progress for a suitable time period such as for instance between 20 minutes and 1 hour at around 37°.

One of the key advantages of the developed technique for producing microbeads based on natural-derived polymers is the possibility to embed therein bioactive molecules for any suitable use in a homogeneous manner. When it comes to natural-derived polymers, incorporation of bioactive molecules or cells into micro-beads during fabrication is a difficult task, particularly due to harsh microbeads manufacturing conditions not allowing it. For instance, pure fibrin micro-beads or nanoparticles described in literature are prepared using an oil-emulsion technique including heating to 60-80 °C (Gorodetsky R 1999) (Gerard Marx 2002). Moreover, previously developed techniques focused on covalently or physically bind of bioactive molecules within fibrin matrices (Hubbell JA 2002). On the other hand, fabrication of natural polymer micro-beads under mild conditions as described in the present disclosure permits to bioactive molecules and/or cells to be added to e.g. the catalysers and/or precursors solutions into the sample reservoirs of the microfluidic chip, without negative consequences on their bioactivity or viability, respectively, and thus being incorporated within natural-derived polymers micro-beads.

In some embodiments, therefore, the method is characterized in that at least one sample reservoir further comprises a bioactive molecule as the ones previously described. Alternatively or additionally, the microfluidic chip can further comprise at least one more sample reservoir comprising only a bioactive molecule and no polymer material precursor or catalyser. Such a reservoir will be referred to herein also as "bioactive molecule reservoir". A sketch of this embodiment of the chip of the invention is shown on Figure 1.

In embodiments where the use of a bioactive molecule is contemplated, the method is characterized in that the temperature of polymerization of the polymeric material does not alter the physico-chemical properties or the activity of the bioactive molecule.

As anticipated, one of the big advantages of the method of the invention relies in the possibility of embedding bioactive molecule within a microbead in a homogeneous manner. This is due to the very nature of the microfluidic chip in particular, especially in cases where a bioactive molecule is already included in one or both of the sample reservoirs. Once the contents of said reservoirs get in touch into the chip's mixing point, they start to fuse and merge so to create a uniform polymeric mixture that eventually permits the homogeneous distribution of all the components inside the microbeads. A homogenous distribution of bioactive molecules within microbeads, *inter alia,* increases the range of compounds' dosage that can be loaded into a bead (i.e. coupling bioactive molecules to the microbeads' surface is more limited due to the available surface), protects more efficiently the bioactive molecules and prolongs the release of the bioactive molecules, particularly when used in a tissue engineering scenario. The method of the invention thus facilitates and simplifies the achievement of functional compounds-loaded natural-derived polymer microbeads. Nevertheless, the microbeads of the invention can be further functionalized in their core and/or surface with any further suitable active agent as those previously listed, with any suitable means.

### EXAMPLES

To describe and illustrate more clearly the present invention, the following examples are provided in detail, which however are not intended to be limiting of the invention.

Stress urinary incontinence (SUI), involuntarily loss of urine, is one of the top ten most common medical conditions affecting adult women. The primary cause of SUI is the relaxation of the pelvic floor, increased abdominal pressure, or trauma caused from childbirth or infrequent bowel movements. Bulking agent injections, providing mechanical support to urinary tract tissues, is widely employed treatment option for patients with these conditions. For an effective treatment of this kind of condition, an ideal bulking agent should be biocompatible, non-immunogenic, and cause minimal fibrosis at the injection site; additionally, it should also trigger neo-host tissue regeneration around the urethra.

The present examples describe a novel injectable biomaterial made of collagen as a carrier material and fibrin beads loaded with recombinant insulin-like growth factor 1 (α₂PI₁₋₈-MMP-IGF-1) for short-term bulking effect and long-term functional muscle regeneration. The ultimate aim of this experimental setting was to trigger long-term functionality of urinary tissue regeneration by promoting host smooth muscle cell migration to the injection site through cell-mediated and sustained delivery of α₂PI₁₋₈-MMP-IGF-1. Fibrin is a promising starting material due to its inherited integrin and growth factor binding sites. Human fibrin glue is a routinely utilized material as complement for surgical sutures and as a support material in reconstructive urological surgery. Besides, fibrin glue can be utilized to restore closure of urinary fistula by promoting host fibroblast proliferation, resulting in connective tissue augmentation. On the other hands, micro/nano-particles suspended in a biodegradable carrier material might offer a promising option for the formulation of injectable bulking agents.

Beads were produced using a microfluidics platform according to the invention and were then embedded into collagen gels. The microfluidic chip allowed optimal control over size, shape, and biological properties of the beads compared to conventional oil emulsion methods. The so-obtained fibrin beads were analysed in regard of their morphology, their growth factor binding efficiency, their stability in the presence of cells as well as their biocompatibility. The in vitro characterization of the new bulking formulation included rheological measurements as well as growth factor release and its effect on urinary tract smooth muscle cells.

### Example 1: Preparation of fibrin beads

Fibrin beads were obtained by modifying a flow-focusing microfluidic system previously described in an article by Allazetta et al. (Allazetta S 2013), the content of which is incorporated herein by reference in its entirety. Computer-controlled syringe pumps (neMESYS from Cetoni, Germany) were used to adjust flow rates. Briefly, one microfluidic channel was loaded with 40 mg/mL of human fibrinogen (plasminogen, fibronectin-depleted; Enzyme Research Laboratories, South Bend, IN, USA) at a flow rate of 1.5 µl/min and the second channel was loaded with an enzyme solution containing 200 U/mL human thrombin (Sigma Aldrich, Switzerland), 200 U/mL factor XIIIa (Fibrogammin, CSL Behring UK) and 10 mM Ca2+ in tris-buffered saline (TBS) at a flow rate of 1 µl/min. For the fabrication of bioactive fibrin beads, the enzyme solution was supplemented with a recombinant IGF-1 (α₂PI₁₋₈-MMP-IGF-1). The fibrinogen and enzyme solution were injected into an oil phase of 2% (w/v) hexadecane and 2% (w/v) silicone-based ABIL EM surfactant. The final concentration of fibrin beads was 22 mg/mL. After the production, the fibrin beads were incubated at 37°C for 20 minutes in order to allow their full polymerization. They were then washed several times in PBS using 70 µm cell strainers (BD Biosciences, USA) to remove the oil phase.

### Example 2: Morphological characterization of fibrin beads

The surface morphology of fibrin beads was studied using Scanning Electron Microscopy (SEM) (Figure 2A) as described below. The obtained fibrin beads were very homogeneous in terms of size and porosity, showing highly dense fiber bundling (Figure 2B). The average diameter of the fibrin beads was around 140 µm as determined by bright-field microscopy and image analysis using the software ImageJ (Figure 2C). Fibrin beads were analyzed for their functional groups via FT-IR analysis. The FT-IR spectrum of fibrinogen demonstrated its characteristic amide absorption bands at around 1643 cm-1, 1550 cm-1, and 1240 cm-1, which are indicative of amide I, II, and III groups, respectively (Figure 2D). The same bands for fibrin were observed around 1687 cm-1, 1550 cm-1, and 1276 cm-1. The bands at around 1110 cm-1 can be attributed to C-N stretching (Figure 2D). The absence of characteristic silicone bands around 800 cm-1, 1022 cm-1, and 1100 cm-1 showed the obtained fibrin beads were silicone-free.

Moreover, as shown on Figure 3, the α₂PI₁₋₈-MMP-IGF-1 was homogeneously distributed within the polymeric matrix of the obtained microbeads.

### Morphology, size distribution, and spectrometric characterization of fibrin beads

The morphological characterization was performed using scanning electron microscopy (SEM, XLF30, Philips). The beads were fixed with 1% tannic acid and 1.25% glutaraldehyde, then washed with 0.1 M cacodylate, and dehydrated in increasing ethanol concentrations prior to critical point drying. They were then coated with gold/palladium. The images were obtained with a voltage of 10 kV. The average size of the beads was determined using a bright field microscope (Zeiss, AxioCam). The captured images were analyzed using the ImageJ software. To confirm the chemical structure of fibrin beads and removal of silicon after extensive washing steps, Fourier Transform Infrared (FT-IR) spectroscopy (Spectrum Two, Perkin Elmer, USA) was performed. Therefore, fibrin beads were lyophilized and then powdered prior to measurement. The same amount of pure fibrinogen was used as control. The scanning range was from 4000 to 650 cm-1 with a resolution of 4 cm-1.

### Example 3: α₂PI₁₋₈-MMP-IGF-1 binding efficiency to fibrin beads

The retained amount of α₂PI₁₋₈-MMP-IGF-1 in fibrin beads was compared to the amount of bound growth factor in bulk fibrin gels. Fibrin gels as well as fibrin beads were initially loaded with 25 nM α₂PI₁₋₈-MMP-IGF-1 and their release profiles were determined over 7 days (Figure 4). On the first day, around 12.3 % and 20.3 % of α₂PI₁₋₈-MMP-IGF-1 was released from fibrin gels and fibrin beads, respectively. After the initial release, the release curves quickly plateaued, showing the covalent binding of α₂PI₁₋₈-MMP-IGF-1 to fibrin. After plasmin degradation on day 7, it was shown that around 79% of the initially loaded amount of α₂PI₁₋₈-MMP-IGF-1 was still present within fibrin beads. In contrast, non-conjugated IGF-1 was released rapidly within 2 days from fibrin gels (over 80 % of the total loading amount).

### α₂PI₁₋₈-MMP-IGF-1 binding efficiency, to fibrin beads

The α₂PI₁₋₈-MMP-IGF-1 binding to fibrin beads was evaluated by its release pattern over 7 days under non-enzymatic condition. 100 µl of fibrin bead solution, conjugated with 25 nM of α₂PI₁₋₈-MMP-IGF-1 were incubated in PBS supplemented with 0.1% BSA and 1% penicillin/streptomycin for 7 days. 100 µl of fibrin gels, containing either 25 nM of α₂PI₁₋₈-MMP-IGF-1 or 25 nM of wild type (wt) IGF-1, were prepared as controls. At day 7, fibrin beads and fibrin gels were degraded using 2 U/mL of plasmin (Roche) to determine the remaining amount of growth factor within the constructs. The amount of the released α₂PI₁₋₈-MMP-IGF-1 and wt IGF-1 in the daily collected release buffer was quantified using a human IGF-1 DuoSet Elisa Kit (R&D systems, CH). The cumulative release data of the samples was normalized to the total amount of loaded growth factor in the fibrin beads and fibrin gels, and plotted as percentage.

### Example 4: Evaluation of fibrin bead degradation in vitro

Fibrin beads were added to hSMCs seeded 12 well-plates and cultured for 7 days to determine their degradation behaviour in the presence of cells as described below. At different time points fibrin beads were visualized under a bright-file microscope and the number and the diameter of non-degraded beads was determined (Figure 5A and 5B). In vitro degradation results demonstrated that 93% of fibrin beads were degraded after 4 days of incubation with hSMCs (Figure 5A). However, fibrin gels, having the same protein concentration as the fibrin bead samples, showed a considerably higher mass loss after 24 hours. Fibrin gels lost around 87% of their initial mass within the first day when incubated with cells (data not shown). In contrast, the average diameter of non-degraded fibrin beads did not change significantly at any time point (Figure 5B).

### Cell culture

Human smooth muscle cells (hSMCs) were extracted from human ureter biopsies, and cultured as previously described (Engelhardt E-M 2010). All in vitro experiments were conducted with hSMCs between passage 6 and 7. hSMCs were cultured in minimum essential alpha medium (α-MEM), supplemented with 10% fetal bovine serum (FBS), and 1% penicillin/streptomycin (α-MEM + 10% FBS). If not otherwise stated, all reagents were purchased from Gibco (Carlsbad, CA).

### In vitro stability of fibrin beads

To determine the effect of cells on bead degradation, hSMCs were seeded at 80,000 cells/well in 12-well plates. They were allowed to attach in an incubator at 37°C for 3 hours. 10 µl (30 mg/mL) of fibrin beads were then added to each well and they were placed at 37°C for 7 days. 75 µl of fibrin gels (4 mg/mL) were used as controls. The number of undegraded beads and their diameters were determined at several time points using a bright-field microscope (Zeiss, AxioCam).

### Example 5: Proliferation, viability, migration, and immunostaining of hSMCs in the presence of fibrin beads

hSMCs proliferation in the presence of fibrin beads was evaluated using an AlamarBlue assay as described below. Cells were cultured in sfα-MEM in the presence of fibrin beads with or without conjugated α₂PI₁₋₈-MMP-IGF-1. As control groups, cells were also cultured in α-MEM + 10% FBS (positive control) and sfα-MEM only (negative control). The measured AlamarBlue-specific fluorescence was converted into cell numbers using the standard curve, obtained from the known number of cells at each time point. After 3 days in culture, no significant change in cell number was obtained under the different conditions, except for the negative control samples (Figure 6A). A 2.80-fold increase in hSMCs cell number was seen in the presence α₂PI₁₋₈-MMP-IGF-1 conjugated fibrin beads, whereas only a 2.41-fold increase was observed in the presence of fibrin beads without conjugated growth factor. Cell viability was above 89 % in all samples on day 3. Cell attachment and migration to fibrin beads were also observed on days 1 and 3 (Figure 6B) The trans well migration assay performed with GFP-expressing hSMCs showed that cell migration started 2 hours after incubation (Figure 7). After 8 hours, a significantly greater number of hSMCs had migrated towards the compartment containing α₂PI₁₋₈-MMP-IGF-1 loaded fibrin beads compared to the number of hSMCs that had migrated towards the compartment containing fibrin beads without growth factor (***p<0.001). The presence of some specific hSMC markers was confirmed by immunohistochemistry. The staining was performed on cells that were cultured in the presence of fibrin beads for 3 days. As seen in Figure 8, cells expressed α-SMA, and COL1A1, two proteins that are responsible for the elasticity of the urinary tissue. However, no smoothelin expression was observed under any conditions.

### In vitro biocompatibility of fibrin beads

The change in metabolic activity of hSMCs in the presence of fibrin beads with or without growth factor was evaluated using AlamarBlue assay. hSMCs at a concentration of 50,000 cells/mL were seeded into 12 well-plates and allowed to attach for 3 hours. 100 µl of fibrin beads containing either 25 nM of α₂PI₁₋₈-MMP-IGF-1 or no growth factor were added to each well being filled with α-MEM supplemented with 0.1 % FBS. Cells cultured either in α-MEM + 10% FBS or sfα-MEM were used as positive control and negative control respectively. AlamarBlue-specific fluorescence was measured with a microplate reader (Infinite M200, Tecan, CH) at an excitation wavelength of 560 nm and an emission wavelength of 590 nm over 3 days. A standard curve, obtained from a known number of hSMCs, was used to link the increasing AlamarBlue-specific fluorescence to cell proliferation. Viability of hSMCs in the presence of fibrin beads with/out growth factor was evaluated using Live/Dead staining (Live/Dead®Viability/Cytotoxicity Kit for mammalian cells, Invitrogen) on day 1 and 3 after bead addition. Images were taken with a Zeiss Axioplan microscope. For cell migration studies, hSMCs stably expressed the green fluorescent protein (GFP). The migration of GFP-labeled-hSMCs was monitored with a Cell IQ imaging system (Cambridge) for 15 hours. Therefore, hSMCs were loaded on the upper side of the membrane in FBS-free α-MEM (sfα-MEM). Either sfα-MEM, or sfα-MEM containing 100 µl of fibrin beads loaded with 25 nM of α₂PI₁₋₈-MMP-IGF-1 were loaded on the bottom side of the well plate. Sfα-MEM supplemented with 1% FBS was used as positive control. Cell migration data was evaluated using the Cell IQ Analyzer software. The differentiation of hSMCs in the presence of fibrin beads with or without growth factor was evaluated by immunohistochemistry. Cells were fixed and incubated with primary antibodies for smooth muscle alpha-actin (α-SMA) (1:100, Abcam, Cambridge, UK), smoothelin (SMTH) (1:250, Abcam, Cambridge, UK) or collagen type I (COL1A1) (1:250, Abcam, Cambridge, UK) at 4°C overnight. The day after, corresponding secondary anti-mouse antibodies (Abcam, Cambridge, UK) were added for 1 h. Nuclei were stained with DAPI. Immunostained samples were visualized under a fluorescence microscope (Zeiss AxioPlan) and further processed using the software Fiji.

### Example 6: Preparation of collagen gels containing fibrin beads (Cf_b gels)

Cylindrical collagen gels were prepared by neutralization of 1 mL of sterile rat-tail type I collagen (2.16 mg/mL, First Link, UK) and 0.1 mL of 10X-concentrated Dulbecco's Modified Eagle's Medium with 1 M sodium hydroxide. 100 µL of fibrin bead solution, containing either 25 nM of α₂PI₁₋₈-MMP-IGF-1 or no growth factor was added to the collagen solution after neutralization. The neutralized solution was cast into cylindrical molds (6 mm diameter x 2 mm height). Molds were then incubated at 37°C for 30 min to allow complete collagen gelation. Cellular gels were prepared by adding 250.000 cells/gel simultaneously with fibrin bead solution. Cellular collagen gels without fibrin beads were prepared as controls.

### Example 7: Rheological properties of Cf_b gels

To determine the rheological behavior of collagen gels and Cf_b gels, a neutralized collagen solution was placed on the metal rheometer plate as described below. The viscous modulus (G") and the storage modulus (G') were measured at an oscillatory frequency of 1 Hz at 37°C. Within the first 3 min of the measurement, G" values of collagen and Cf_b gels increased up to an average of 4.6 and 9.5 times of their original values, respectively (Figure 9A). For both gels, gelation started 29 ± 5.87 seconds after the start of the measurement. After 13 minutes, collagen and Cf_b gels showed a sharp increase in G' values from 39.44 Pa to 59.76 Pa and 30.34 Pa to 48.86 Pa, respectively (Figure 9B).

G" and G' values of the gels were also evaluated as a function of changing oscillation frequency. Over a time frame of 10 min, the frequency was increased from 0.1 Hz to 10 Hz. (Figure 9C and 9D). G" values of both collagen and Cf_b gels showed frequency dependent behavior in contrast to Deflux that showed almost no response to the frequency change (Figure 9C). The highest G" values of collagen, Cf_b gels, and Deflux were determined as 312.54 ± 14.91 Pa, 396.65 ± 21.42 Pa, and 659.54 ± 29.51 Pa, respectively. Collagen gels had a maximum G' value of 533.55 ± 18.381 Pa, while Cf_b gels had a maximum one of 423.98 ± 12.67 Pa at 10 Hz. (Figure 9D). Deflux had the highest G' value with 1948.45 ± 149.67 Pa at the same frequency (Figure 9D).

### Experimental setting

Rheological measurements were performed to determine the storage (elastic) (G') and loss (viscous) (G") modulus values of collagen fibrin bead (Cf_b) gels either at a constant frequency or in function of frequency in oscillatory mode, using a C-VOR rheometer (Bohlin, Germany). The gel point of the samples, where G' and G" modulus values intercept, was determined in single frequency oscillatory mode at 1 Hz at 37°C for 20 min. Therefore, 2 mL of neutralized collagen solution with or without fibrin beads were loaded between, parallel metal plates with 150 µm of gap distance. Samples were allowed to fully gelate under oscillation frequency of 1 Hz.

Frequency sweep tests were also performed in the frequency range from 0.1 to 10 Hz at room temperature for 10 min to evaluate the viscoelasticity behavior of Cf_b gels as compared to collagen gels. Samples were subjected to increasing oscillation frequency using parallel metal plates with a 1.6 mm of gap distance. They were initially compressed to 80 % of their original thickness to avoid slippage. A commercial bulking agent, Deflux, was used as a reference.

### Example 8: Cell proliferation and histological evaluation of Cf_b gels

To investigate the effect of fibrin beads on hSMCs proliferation, both, cells and fibrin beads with or without conjugated α₂PI₁₋₈-MMP-IGF-1, were added to the neutralized collagen solution prior to gelation. Three and seven days after gel preparation, AlamarBlue-specific fluorescence of the constructs was measured. An established standard curve allowed converting the AlamarBlue-specific fluorescence into a cell number. On day 3 and 7, no significant difference was found in cell proliferation between fibrin bead loaded collagen and collagen gels (Figure 10A). The size of the fibrin beads was well preserved after embedding them into collagen gels in the absence of cells (Figure 10B and 10C). HE staining of the samples showed that some of the beads were swollen at day 1 and 3, increased their volume up 1.5 times of their original volume in cell culture conditions (Figure 10C). Furthermore, hSMCs attachment to the surface of the fibrin beads was also clear on day 7, shown by arrows (Figure 10D).

### Experimental setting

AlamarBlue assay was used to analyze hSMCs proliferation seeded in 1 mL of Cf_b gels either loaded with 25 nM of α₂PI₁₋₈-MMP-IGF-1 or no growth factor over 7 days. AlamarBlue-specific fluorescence was measured with a microplate reader (Infinite M200, Tecan, Switzerland) at 560 nm excitation and 590 nm emission wavelengths. The number of cells within the samples was calculated using a standard curve, generated using a series of known numbers of hSMC seeded into 1 mL of collagen gels. Acellular and cellular Cf_b gels were embedded in paraffin on days 1 and 7, and then sectioned (thickness of 5 µm). De-paraffinized sections were stained with hematoxylin and eosin (HE).

### Example 9: Injection of collagen-fibrin micro-bead matrix as bulking agent in a rat and rabbit model

5.25 mL bovine collagen (5 mg/mL, Symatese) 0.8 mL of 10x MEM, 1.2 mL alpha-MEM medium, and 100 µL of fibrin micro-beads with or without growth factor were mixed together. Gelation was induced by the addition of 1.070 mL 0.1 M sodium hydroxide. The collagen solution was filled into a syringe having a volume of 1 mL or 3 mL, and was either incubated on ice or at room temperature for 30 min before injection into the bladder wall of a rat or a rabbit. A 25G syringe was used for injection and the tested injection volumes were 0.5 mL, 1 mL and 1.5 mL. The injected collagen volumes were monitored for 3 minutes to ensure the re-gelation of the collagen, before placing back the bladders into the abdominal cavity and closure of the cavity (Figure 11A, B, and C).

### Example 10: Implant of collagen-flbrin scaffold as tissue engineering therapy in a rat model

Collagen gel layers were prepared by neutralization of 3 mL of sterile rat tail type I collagen (2.16 mg/mL, First Link, UK) and 0.8 mL of 10X-concentration of Dulbecco's Modified Eagle's Medium with 1 M sodium hydroxide in square-shaped stainless steel molds (2.5 x 3 x 2.5 cm). After gelation of the first layer of collagen in a 5% CO₂ incubator at 37°C for 20 minutes, fibrin solution, either plain or conjugated with varying concentrations of α₂PI₁₋₈-MMP-IGF-1, were casted onto this layer of collagen gel and subsequently the second layer of already gelled collagen was placed onto the collagen-fibrin bi-layers to form the trilayer construct (Figure 12A and B).

### Citation list

Allazetta S, Hausherr TC, Lutolf MP. "Microfluidic synthesis of cell-type-specific artificial extracellular matrix hydrogels." Biomacromolecules 12 (2013): 1122-1131.
Bae KH, Yoon JJ, Park TG. "Fabrication of hyaluronic acid hydrogels beads for cell encapsulation." Biotechnol Prog 22 (2006): 297-302.
Brown, R. Cell-indepentent fabrication of tissue equivalents. Patent WO2006/003442A2. July 5, 2005.
Chiu LL, Weisel RD, Li RK, Radisic M. "Defining conditions for covalent immobilization of angiogenic growth factors onto scaffolds for tissue engineering." J Tissue Eng Regen Med 5 (2011): 69-84.
Corcos J, Collet JP, Dyer R. "Multicenter randommized clinical trial comparing surgery and collagen injections for treatment of female stress urinary incontinence." Urology 65 (2005): 898-904.
Cummings CL, Gawlitta D, Nerem RM, Stegemann JP. "Properties of engineered vascular constructs made from collagen, fibrin, and collagen-fibrin mixtures." Biomaterials 25 (2003): 3699-3706.
Engelhardt E-M, Stegberg E, Brown RA, Hubbell JA, Wurm FM, Adam M, Frey P. "Compressed collagen gel: a novel scaffold for human bladder cells." J Tissue Eng Regen Med 2010;4(2):123-130.
Ehrbar M, Zeisberger SM, Raeber GP, Hubbell JA, Schnell C, Zisch AH. "The role of actively released fibrin-conjugated VEGF for VEGF receptor 2 gene activation and the enhancement of angiogenesis." Biomaterials 29 (2008): 1720-1729.
Eyrich D, Brandl F, Appel B, Wiese H, Maier G, Wenzel M, Staudenmaier R, Goepferich A, Blunk T. "Long-term stable fibrin gels for cartilage engineering." Biomaterials 28 (2007): 55-65.
Gerard Marx, Raphael Gorodetsky. Fibrin nanoparticles and uses thereof. Patent WO2003037248A2. August 12, 2002.
Gorodetsky R, Clark RA, An J, Gailit J, Levdansky L, Vexler A, Berman E, Marx G. "Fibrin micro-beads (FMB) as biodegradable carriers for culturing cells and for accelerating wound healing." J Invest Dermatol 112 (1999): 866-872.
Han H, Ao Q, Chen G, Wang S, Zuo H. "A novel basic fibroblast growth factor delivery system fabricated with heparin-incorporated fibrin-fibronectin matrices for repairing rat sciatic nerve disruptions." Biotechnol Lett 32 (2010): 585-591.
He Q, Zhao Y, Chen B, Xiao Z, Zhang J, Chen L, Chen W, Deng F, Dai J. "mproved cellularization and angiogenesis using collagen scaffolds chemically conjugated with vascular endothelial growth factor." Acta Biomater 7 (2011): 1084-1093.
Hubbell JA, Schense J, Zisch A, Hall A. Enzyme-mediated modification of fibrin for tissue engineering. Patent US20020168718A1. November 14, 2002.
Hunter GK. "Chondroitin sulfate-derivatized agarose beads: a new system for studying cation binding to glycosaminoglycans." Anal Biochem 165 (1987): 435-441.
Jeon O, Soo HR, Ji HC, Kim BS. "Control of basic fibroblast growth factor release from fibrin gel with heparin and concentrations of fibrinogen and thrombin." Release 105 (2005): 249-259.
Jockenhoevel S, Zund G, Hoerstrup SP, Chalabi K, Sachweh JS, Demircan L, Messmer BJ, Turina M. "Fibrin-gel advantages of a new scaffold in cardiovascular tissue engineering." Eur J Cardithorac Surg 19 (2011): 424-430.
Kawazoe N, Inoue C, Tateishi T, Chen G. "A cell leakproof PLGA-collagen hybrid scaffold for cartilage tissue engineering." Biotechnol Prog 26 (2010): 819-826.
Kim IG, Oh SH, Lee JY, Lee JH. "Bioactive porous beads as injectable urethral bulking agent: in vivo animal study for the treatment of urinary incontinence." Tissue Eng Part A 17 (2011): 655-664.
Lai VK, Frey CR, Kerandi AM, Lake SP, Tranquillo RT, Barocas VH. "Microstructural and mechanical differences between digested collagen-fibrin co-gels and pure collagen and fibrin gels." Acta Biomater 8 (2012): 4031-4042.
Lightner DJ, Calvosa C, Andersen R, Klimberg I, Brito CG. "A new injectable bulking agent for treatment of stress urinary incontinence: results of a multicenter, randomized, controlled, double-blind study of Durasphere." Urology 58 (2011): 12-15.
Lu H, Oh HH, Kawazoe N, Yamagishi K, Chen G. "PLLA-collagen and PLLA-gelatin hybrid scaffolds with funnel-like porous structure for skin tissue engineering." Sci Technol Adv Mater 13 (2012).
Ma F, Xiao Z, Chen B, Hou X, Dai J, Xu R. "Linear Ordered Collagen Scaffolds Loaded with Collagen-Binding Basic Fibroblast Growth Factor Facilitate Recovery of Sciatic Nerve Injury in Rats ." Tissue Eng Part A 20 (2014): 1253-1262.
Macomber L, Sommerich R, Shenoy V, Halvorsen M. Collagen device and method of preparing the same. Patent EP1561480A2. February 8, 2005.
Martino M, Briquez P, Güc E, Tortelli F, Kilarski WW, Metzger S, Rice JJ, Kuhn GA, Müller R, Swartz MA, Hubbell JA. "Growth Factors Engineered for Super-Affinity to the Extracellular Matrix Enhance Tissue Healing ." Science 343 (2014): 885-888.
Mayer RD, Dmochowski RR, Appell RA, Sand PK, Klimberg IW. "Multicenter prospective randomized 52-week trial of calcium hydroxylapatite versusu bovine dermal collagen for treatment of stress urinary incontinence." Urology 69 (2007): 876-880.
Mullen LM, Best SM, Brooks RA, Ghose S, Gwynne JH, Wardale J, Rushton N, Cameron RE. "Binding and release characteristics of insulin-like growth factor-1 from a collagen-glycosaminoglycan scaffold." Tissue Eng Part C Methods 16 (2010): 1439-1448.
Niger C, Beazley KE, Nurminskaya M. "Induction of chondrogenic differentiation in mesenchymal stem cells by TGF-beta cross-linked to collagen-PLLA scaffold by transglutaminase." Biotechnol Lett 35 (2013): 2193-2199.
Oh SH, Bae JW, Kang JG, Kim IG, Son JY, Lee JY, Park KD, Lee JH. "Dual growth factor-loaded in situ gel-forming bulking agent: passive and bioactive effects for the treatment of urinary incontinence." J Mater Sci: Mater Med 26 (2015): 5365-5376.
Page RL, Malcuit C, Vilner L, Voijtic I, Shaw S, Hedblom E, Hu J, Pins GD, Rolle MW, Dominko T. "Restoration of skeletal muscle defects with adult human cells delivered on fibrin microthreads." Tissue Eng Part A 17 (2011): 2629-2640.
Park YK, Tu TY, Lim SH, Clement IJ, Yang SY, Kamm RD. "In vitro microvessel growth and remodeling within a three-dimensional microfluidic environment." Cell Mol Bioeng 7 (2014): 15-25.
Perka C, Arnold U, Spitzer RS, Lindenhayn K. "The use of fibrin beads for tissue engineering and subsequential transplantation." Tissue Eng 7 (2001): 359-361.
Peterson AW, Caldwell DJ, Rioja AY, Rao RR, Putnam AJ, Stegemann JP. "Vasculogenesis and angiogenesis in modular collagen-fibrin microtissues." Niomater Sci 2 (2014): 1497-1508.
Rao RR, Peterson AW, Ceccarelli J, Putnam AJ, Stegemann JP. "Matrix composition regulates three dimensional network formation by endothelial cells and mesenchymal stem cells in collagen/fibrin materials." Angiogenesis 15 (2012): 253-264.
Rowe SL, Stegemann JP. "Microstructure and mechanics of collagen-fibrin matrices polymerized using ancrod snake venom enzyme." J Biomech Eng 131 (2009).
Sacchi V, Mittermayr R, Hartinger J, Martino MM, Lorentz KM, Wolbank S, Hofmann A, Largo RA, Marschall JS, Groppa E, Gianni-Barrera R, Ehrbar M, Hubbell JA, Redl H, Banfi A. "Long-lasting fibrin matrices ensure stable and functional angiogenesis by highly tunable, sustained delivery of recombinant VEGF164." Proc Natl Acad Sci 111 (2014): 6952-6957.
Sakiyama-Elbert SE, Panitch A, Hubbell JA. "Development of growth factor fusion proteins for cell-triggered drug delivery." FASEB J 15 (2001): 1300-1302.
Schumacher B, Pecher P, Von Specht BU, Stegmann T. "Induction of neoangiogenesis in ischemic myocardium by human growth factors: first clinical results of a new treatment of coronary heart disease." Circulation 97 (1998): 645-650.
Shimizu, Y. Collagen material and process for producing the same. Patent EP1098024A1. June 7, 1999.
Simons M, et al. "Pharmacological treatment of coronary artery disease with recombinant fibroblast growth factor-2:double-blind, randomized, controlled clinical trial." Circulation 105 (2002): 788-793.
Sun B, Chen B, Zhao Y, Sun W, Chen K, Zhang J, Wei Z, Xiao Z, Dai J. "Crosslinking heparin to collagen scaffolds for the delivery of human platelet-derived growth factor." J Biomed Mater Res B Appl Biomater 91 (2009): 366-372.
Tan Q, Chen B, Yan X, Lin Y, Xiao Z, Hou X, Dai J. "Promotion of diabetic wound healing by collagne scaffold with collagen binding vascular endothelial growth factor in a diabetic rat model." J Tissue Eng Regen Med 8 (2014): 195-201.
Tsai SW, Chen CC, Liou HM, Hsu FY. "Preparation and characterization of microspheres comrised of collagen, chondroitin sulfate, and apatite as carriers for osteoblast-like cell MG63." J Biiomed Mater Res A 93 (2010): 115-122.
Voge CM, Kariolis M, MacDonald RA, Stegemann JP. "Directional conductivity in SWNT-collagen-fibrin composite biomaterials through strain-induced matrix alignment." J Biomed Mater Res A 86 (2008): 269-727.
Willerth SM, Arendas KJ, Gottlieb DI, Sakiyama-Elbert SE. "Optimization of fibrin scaffolds for differentiation scaffolds for differentiation of murine embryonic stem cells into neural lineage cells." Biomaterials 27 (2006): 5990-6003.
Wu JM, Xu YY, Li ZH, Yuan XY, Wang PF, Zhang XZ, Liu YQ, Guan J, Guo Y, Li RX, Zhang H. "Heparin-functionalized collagen matrices with controlled release of basic fibroblast growth factor." J Mater Sci Mater Med 22 (2011): 107-114.
Zhao W, Han Q, Lin H, Sun W, Gao Y, Zhao Y, Wang B, Wang X, Chen B, Xiao Z, Dai J. "Human basic fibroblast growth factor fused with Kringle4 peptide binds to a fibrin scaffold and enhances angiogenesis." Tissue Eng A 15 (2009): 991-998.
Zhou H, Xu HH. "The fast release of stem cells from alginate-fibrin microbeads in injectable scaffolds for bone tissue engineering." Biomaterials 32 (2011): 7503-7513.

## Claims

1. A scaffold material for use in tissue engineering, **characterized in that** it comprises a plurality of polymeric microbeads embedded within a polymeric carrier, wherein the microbeads and the carrier are substantially composed of the same or different natural polymeric material or extracellular matrix-derived polymeric material.

2. The scaffold material of claim 1, **characterized in that** the microbeads have a diameter comprised between 10 and 1000 µm, more preferably between 80 and 500 µm, even more preferably between 100 and 200 µm.

3. The scaffold material of claims 1 or 2, **characterized in that** the average molecular weight of the polymeric material substantially composing the microbeads and/or the carrier is comprised between about 1 and 1000 kDa, preferably between 50 and 600 kDa.

4. The scaffold material of any previous claim, **characterized in that** the microbeads volume constitutes in between 1 to 99 % of the volume of the scaffold material, preferably between 20 to 60 %.

5. The scaffold material of any previous claim, **characterized in that** it is flowable and injectable through a cannula or a needle.

6. The scaffold material of any previous claim, **characterized in that** the microbeads comprise a bioactive molecule homogeneously embedded therein.

7. The scaffold material of claim 6, **characterized in that** the bioactive molecule is released from the microbeads upon degradation of this latter in a substantially linear fashion.

8. The scaffold material of any previous claim, **characterized in that** the polymeric carrier is substantially composed of collagen and the microbeads are substantially composed of fibrin.

9. The scaffold material of claims 1 to 8 for use in the treatment or prevention of a pathological condition in a subject.

10. A method of manufacturing microbeads substantially composed of a natural polymeric material or an extracellular matrix-derived polymeric material, wherein the polymerization of said polymeric material is a temperature-dependent polymerization, said method comprising the steps of:
a) providing a microfluidic chip comprising:
- at least two sample reservoirs operatively connected with a pressure source adapted to apply a positive pressure thereon, at least one of said reservoirs being intended for containing an aqueous solution comprising a precursor of the polymeric material substantially composing the microbeads and at least one of said reservoirs being intended for containing an aqueous solution comprising a polymerization catalyser;
- at least one channel operatively connected with each of the said sample reservoirs through their inlets;
- a mixing point operatively connecting the outlets of each of said channels;
- at least one microsized channel stemming from said mixing point;
- at least one organic phase reservoir operatively connected with a pressure source adapted to apply a positive pressure thereon, said reservoir being intended for containing an organic solution;
- at least one microsized channel operatively connected with each of the said organic phase reservoirs through its inlet, this microsized channel intersecting the microsized channel stemming from the mixing point in a beads-forming point;
- a focusing element stemming from said beads-forming point adapted so to canalize the microbeads; and
- a microbeads reservoir operatively connected with both the focusing element and means for regulating the temperature in the reservoir;
b) providing a precursor of the polymeric material substantially composing the microbeads into at least one of the sample reservoirs and a polymerization catalyser into at least one of the sample reservoirs;
c) applying a positive pressure on the sample reservoirs so that the precursor of the polymeric material and the catalyser are allowed to flow into the channels operatively connected therewith and to mix in the mixing point;
d) applying a positive pressure on the organic phase reservoirs so that the organic solution is allowed to flow into the microsized channel operatively connected therewith;
e) collecting the precursor microbeads obtained through the steps a) to d) into the microbeads reservoir;
f) regulating the temperature in the microbeads reservoir; and
g) let the precursor microbeads into the reservoir for a sufficient time for permitting a temperature-dependent polymerization thereof,
wherein step c) and step d) are interchangeable.

11. The method of claim 10, **characterized in that** the microfluidic chip is a T-junction, Y-junction or flow focusing microfluidic chip.

12. The method of claims 10 or 11, **characterized in that** the precursor of the polymeric material is functionalized.

13. The method of claims 10 to 12, **characterized in that** at least one sample reservoir further comprises a bioactive molecule.

14. The method of claim 13, **characterized in that** the bioactive molecule is eventually homogeneously embedded into the microbeads.

15. The method of claims 10 to 14, **characterized in that** the polymeric material precursor is fibrinogen and the catalyser is a mixture of thrombin and Factor XIIIa.

16. Microbeads obtained through the method of claims 10 to 15.
